(19) Europäisches Patentamt
European Patent Office
Office européen des brevets

(11) EP 4 706 772 A2

(12) EUROPEAN PATENT APPLICATION

(43) Date of publication:
**11.03.2026 Bulletin 2026/11**

(21) Application number: **25223781.3**

(22) Date of filing: **27.02.2023**

(51) International Patent Classification (IPC):
***A61P 35/00*** *(2006.01)*

(52) Cooperative Patent Classification (CPC):
**C07D 231/14; A61P 35/00**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **28.02.2022 EP 22382169**

(62) Document number(s) of the earlier application(s) in accordance with Art. 76 EPC:
**23706624.6 / 4 486 727**

(71) Applicant: **Química Sintética, S.A.**
**28805 Alcalá de Henares (ES)**

(72) Inventors:
- **BARRECA, Giuseppe**
  **23896 SIRTORI (Lc) (IT)**
- **PARAVIDINO, Piero**
  **20018 SEDRIANO (IT)**
- **CARCONE, Luca**
  **20151 MILANO (IT)**
- **ZAMPIERI, Massimo**
  **20811 CESANO MADERNO (IT)**
- **PENGO, Daniele**
  **20037 PADERNO DUGNANO (IT)**
- **RONZONI, Silvano**
  **20148 MILANO (IT)**
- **PESENTI, Cristina**
  **21055 GORLA MINORE (IT)**
- **COPPOLA, Marianna**
  **13100 VERCELLI (IT)**
- **MASCIOCCHI, Norberto**
  **22100 COMO (IT)**

(74) Representative: **ZBM Patents - Zea, Barlocci & Markvardsen**
**Rambla de Catalunya, 123**
**08008 Barcelona (ES)**

# (54) CRYSTALLINE FORM OF DAROLUTAMIDE

(57) Crystalline form of darolutamide

The present invention relates to a crystalline form of darolutamide and to the process for its preparation. Furthermore, the invention relates to the use of said crystalline form for the preparation of a pharmaceutical composition useful for treating prostate cancer.

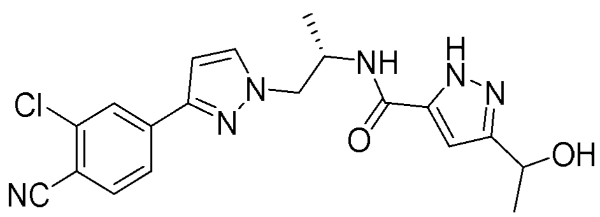

EP 4 706 772 A2

## Description

### Field of the invention

The present invention relates to a new crystalline form of darolutamide, herein called NC form, and to the process for its preparation. Furthermore, the invention relates to a pharmaceutical composition comprising said new crystalline form and one or more acceptable excipients.

### Background art

Darolutamide, chemically also known as N-[(2S)-1-[3-(3-chloro-4-cyanophenyl)-1H-pyrazol-1-yl]-propan-2-yl]-5-(1-hydroxyethyl)-1H-pyrazole-3-carboxamide, is the active pharmaceutical ingredient present in the medicament Nubeqa®, approved by the regulatory authorities in USA, in the European Union and in Australia, for the treatment of nonmetastatic castration-resistant prostate cancer (CRPC).

It can be represented by the following chemical structure as shown in Formula (I).

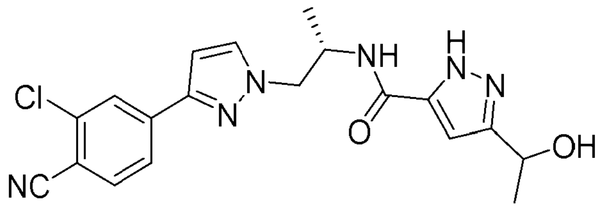

Formula (I)

The structure depicted in formula (I) refers to N-[(2S)-1-[3-(3-chloro-4-cyanophenyl)-1H-pyrazol-1-yl]-propan-2-yl]-5-(1-hydroxyethyl)-1H-pyrazole-3-carboxamide and its tautomer namely N-[(2S)-1-[3-(3-chloro-4-cyanophenyl)-1H-pyrazol-1-yl]-propan-2-yl]-3-(1-hydroxyethyl)-1H-pyrazole-5-carboxamide, as recognized by the skilled person in the art, because of the hydrogen atom of the pyrazole ring may exist in tautomeric equilibrium between the 1- and 2-position.

Darolutamide comprises a mixture of two pharmacologically active diastereomers in a molar ratio 1:1 having the following chemical structure as shown in formula (Ia) and (Ib):

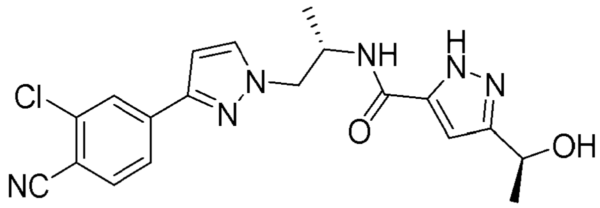

Formula (Ia)

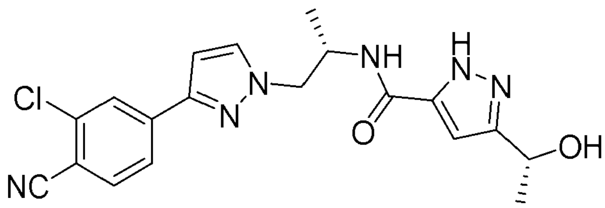

Formula (Ib)

The two diastereomers interconvert in vivo to ketodarolutamide, the primary pharmacologically active metabolite of darolutamide, represented by the chemical structure as depicted in Formula (II) and namely 3-acetyl-N-[(2S)-1-[3-(3-chloro-4-cyanophenyl)pyrazol-1-yl]propan-2-yl]-1H-pyrazole-5-carboxamide.

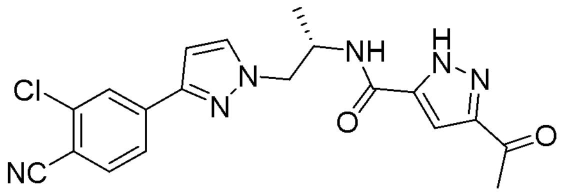

Formula (II)

Darolutamide and its preparation were first disclosed in WO 2011/051540 filed by Bayer AG.

WO 2016/120530 to Bayer AG discloses crystalline Form I, the form used to prepare the final medicament, as reported in figure 11.

In addition, this patent application describes Form I' and Form I", which are the polymorphic forms referred respectively to diastereomers of formula Ia and Ib. As mentioned above these polymorphic forms are not useful to obtain the final medicament, because Darolutamide medicament contains a mixture 1:1 of each diastereomers.

WO 2018/162793 to Bayer AG discloses crystalline particles of darolutamide which have better properties for subsequent formulation process: said particles present a specific surface area (SSA) in the range of 8-16 $m^2/g$ (measured by BET), preferably in the range of 10-15 $m^2/g$ and a round shape. The polymorphic form of said crystalline rounded particles has not been completely characterized in WO 2018/162793: the patent application discloses that they are at least partly in crystalline form I. Furthermore, in WO 2018/162793 the applicant discloses that powder consisting of darolutamide in crystalline Form I, obtained following the teaching of WO 2016/120530, is composed by heterogenous particles with undefined morphology, which means that it is challenging to formulate darolutamide in said polymorphic form, particularly in an example provided during prosecution of EP 3592732 is shown that crystalline particles of Example 1 of WO 2016/120530 show a SSA of 22.1 $m^2/g$.

Document EP 3495352 A1 discloses Form I of darolutamide before and after grinding. Grinding is a common method to reduce particle size and thus generally increase the specific surface area to impart better dissolution rate and bioavailability to a compound. In Example 13 of EP 3495352 A1 the applicant shows that crystallinity of Form I prepared according to WO 2016/120530 decreases significantly after grinding, as supported by an XRPD pattern shown in Fig.12, which shows the same peaks of Form I at much lower intensity and the simultaneous presumed presence of amorphous material.

Darolutamide is formulated as film-coated, immediate-release 300 mg tablets for oral administration: it is given in doses of 600 mg (2 tablets of 300 mg) twice daily with food, equivalent to a total daily dose of 1200 mg. Said high doses are required in order to reach therapeutic plasma concentrations after oral administration, because darolutamide, classified in the Biopharmaceutics Classification System (BCS) as a class II medicament, is a poorly soluble drug in the physiological pH range. Higher doses of active ingredient correspond to higher side effects for the patients.

Thus, it is clear that it would be preferable to obtain a new crystalline form of darolutamide with improved chemical and physical properties in order to reduce the daily dose. This entails a reduction of pill burden and of side effects for patient, resulting in an improvement of the therapeutic treatment.

## Summary of the invention

It is thus a main aspect of the present invention to provide a new crystalline form of darolutamide, herein called Form NC, with improved properties: as for example with good chemical and physical stability, high solubility, enhanced bioavailability and good processability.

Another aspect of the invention is to provide a process to prepare darolutamide in said new crystalline form.

Yet another aspect of the invention is to use said new darolutamide form to prepare formulation.

Still another aspect of the invention is to provide tablets, preferably film coated tablets, containing said new darolutamide form and at least one pharmaceutically acceptable excipient.

## Brief description of the drawings

Figure 1 shows an exemplary X-ray powder diffractogram (XRPD) of Form NC of darolutamide.

Figure 2 shows an exemplary differential scanning calorimetry (DSC) curve of Form NC of darolutamide (30 - 250 °C; 10°C/min).

Figure 3 shows the $^{13}C$ CPMAS NMR spectrum of Form NC of darolutamide.

Figure 4 shows the infrared spectrum (IR) of Form NC of darolutamide.

Figure 5 shows a scanning electron microscopic (SEM) image of Form NC of darolutamide.
(magnification: x 20000, scale bar: 1 micrometer).
Figure 6 shows the calibration curve of SSA measurements.
Figure 7 shows the calibration curve of solubility determination of darolutamide samples.
Figure 8 shows two time-dependent solubility curves of Form NC of darolutamide versus Form I and crystalline rounded particles respectively measured at pH 1 and 7.5 at 37°C.
The x-axis shows the time in minutes (min), the y-axis shows the concentration of darolutamide in solution in (mg/mL).
Figure 9 shows an example of profile fitting (PF) plot with a "split pseudo-Voigt" description for the slightly asymmetric peak shapes related to the peaks falling near about 8.6 and 10.4 degrees 2-Theta of Form NC of darolutamide.
Figure 10 shows the overlay of X-ray powder diffraction profiles (XRPD) referred to Form NC of darolutamide during the stability studies at 40°C and 75% RH, acquired at time zero and after timepoints of 1 month, 3 months, 6 months and 12 months as described in Example 20.
Figure 11 shows XRPD of Darolutamide Form I vs Nubeqa® 300mg.
Figure 12 shows an overlay of the XRPD traces of Form I before and after grinding taken from EP 3 495 352 A1, and Form NC.
Fig. 13 shows an overlay of the XRPD traces of a Form I sample and two ground samples thereof obtained respectively from Comparative Examples 1a (Form I obtained according to WO 2016/120530), 1b and 1c (respectively, manually ground according to EP 3 495 352 A1, and ground in a milling equipment)

## Detailed description of the invention

All terms used in this application, unless otherwise specified, are to be understood in their ordinary meaning as known in the technical field.

The term "nanocrystalline form of N-[(2S)-1-[3-(3-chloro-4-cyanophenyl)-1H-pyrazol-1-yl]-propan-2-yl]-5-(1-hydroxyethyl)-1H-pyrazole-3-carboxamide" or "nanocrystalline form of darolutamide" or "nanocrystalline form" or "nanocrystalline darolutamide" or "NC form" or "Form NC" as used interchangeably herein, refers to a solid crystalline form of darolutamide, wherein each particle of said form is composed by crystallites having a size below 100 nm. Nanocrystalline materials are defined in the art e.g. as: ultrafine-grained single-phase or multiphase polycrystals with grain sizes in the range of 1-100 nm (B. Movahedi, "Introductory Chapter: Nanocrystalline Materials", Nanocrystalline Materials, Feb. 2020, doi: 10.5772/intechopen.90255); single-phase or multiphase polycrystalline solids with a crystallite size of only a few nanometers, usually less than 100 nm (Nasrollahzadeh, M. et al., "Chapter 2 - Types of Nanostructures" Interface Science and Technology, Volume 28, 2019, Pages 29-80); or as polycrystalline materials with grain sizes of up to about 100 nm (Suryanarayana, C., "Nanocrystalline Materials", International Materials Reviews, 1995, 40(2):41-64).

The term "crystallite" or "grain" as used herein refers to coherently scattering domain which composes each particle of darolutamide. The crystallite size can be estimated using X-ray pattern diffraction (XRPD). Peak width of XRPD pattern is due to crystallite size and it varies inversely with crystallite size: as the crystallite size gets smaller, the peak gets broader. This broadening can be related to a crystallite size using the Scherrer equation, which is normally applicable for submicrometric sized materials, see e.g. U. Holtzward and N.Gibson 'The Scherrer equation versus the 'Debye-Scherrer equation', Nature Nanotechnology, 2011, 6, 534.

The estimated size visible by scanning electron microscopy (SEM) or transmission electron microscopy (TEM) is different from X-ray based ones, because with microscopy technique aggregates are imaged and averages are built by number-based distributions, while mass-based averaged values are detected by X-ray methods.

The term "broad peaks" as used herein refers to a XRPD pattern typical of nanocrystalline forms, characterized by wide peaks, well beyond the breadth determined essentially by instrumental effects.

The terms "crystalline Form I of darolutamide" or "Form I of darolutamide" or "Form I" as used interchangeably herein, refer to the crystalline form of darolutamide, which is disclosed and also designated as Form I in WO 2016/120530. Form I of darolutamide can be characterized by having a powder X-ray diffractogram comprising reflections at 8.5, 10.4, 16.6, 16.9, and 24.3 $\pm$ 0.15 degrees 2-Theta, when measured at room temperature with Cu-K$\alpha$ radiation having a wavelength of 1.5419 Å, as reported in WO 2016/120530 patent application.

The term "crystalline rounded particles" of darolutamide as used herein refers to crystalline particle of darolutamide having a specific surface area (SSA) in the range of 8-16 $m^2/g$ , preferably 10-15 $m^2/g$, measured by three-point nitrogen adsorption technique based on the Brunauer, Emmett and Teller (BET) theory, and/or a round shape with a large volume median diameter in the range of 100 - 1000 $\mu$m, preferably between 120 - 800 $\mu$m, more preferably between 150 - 750 $\mu$m, measured by laser light diffraction using air dispersion medium and applying Fraunhofer optical model, as obtained following the teaching of WO 2018/162793.

The term "about" includes the range of experimental errors, which can normally occur performing a measurement, e.g. $\pm$ 5% or $\pm$ 2% or $\pm$ 1%. Sometimes, such a range can lie within the experimental error, typical of standard methods used for the measurement and/or determination of a given value or range. Particularly, the measurement

uncertainty for the Multi-Point B.E.T. specific surface area parameter is equal to 15% for a SSA value greater than 2 $m^2$/g, and 20% for a SSA less than 2 $m^2$/g. It is expressed as extended uncertainty obtained by multiplying standard uncertainty by the coverage factor k equal to 2, corresponding to confidence level of about 95%. The uncertainty is related to the method of analysis and obtained as percent value from the analysis of a representative group of samples in the measurement range of the instrument.

The term "room temperature" as used herein refers to a temperature in the range from about 18°C to about 25°C.

The term "$C_5$-$C_7$ linear or branched alkanes" includes pentane, hexane, heptane in a linear or branched structure.

The term "substituted cycloalkane" as used herein refers to a cyclopentane and cyclohexane with one or more alkyl substituents.

The term "antisolvent" as used herein refers to a liquid, which reduces the solubility of darolutamide in a solvent.

The term "any solid forms" as used herein refers to a darolutamide in any crystalline or amorphous forms.

The term "amorphous" refers to a solid form of a compound that is not crystalline. An amorphous compound possesses no long-range order and does not display a definite X-ray diffraction pattern with reflections.

The term "excipient" means any substance contained in the final pharmaceutical form other than the active ingredient and which generally may not be therapeutically effective by itself. Excipients are essential for the administration of the active substance, as they allow to deliver the drug to the target site. Excipients are commonly referred to as raw materials entering into the composition of a pharmaceutical preparation with the aim of, for example, giving a shape, to facilitate administration and preserve the active ingredient. Furthermore, they contribute to characterize the pharmaceutical preparation from the point of view of appearance, stability, biopharmaceutical profile and acceptability by the patient. Disintegrants, lubricants, fillers, binders, flavours and colorants are considered as excipients.

The term "disintegrant" is referred to an agent used in pharmaceutical preparation of tablets, which causes them to disintegrate and release their pharmaceutical active ingredient on contact with moisture.

The term "lubricant" is referred to an agent added in a small quantity to tablet formulations to reduce the friction and improve their preparation process.

The term "filler" is referred to an agent added to tablet formulations to increase the volume of the material which must be formulated, in order to enable easier processing.

The term "binder" is referred to an agent which ensure that tablets can be made with the required mechanical strength.

Unless otherwise indicated, in the context of the present invention the indication that a composition "comprises" other one or more components/elements means that the indicated components/elements must be present and also other components may be present, but are not necessarily present, in the composition, in addition to the ones specifically recited. In other words, the indication that a composition "comprises" one or more components does not exclude that the composition consists of, or consists essentially of, the recited component(s).

Unless otherwise indicated, in the context of the present invention the percentage and amount of a certain component in a composition are to be referred to the weight of said component with respect to the total weight of the composition.

Unless otherwise indicated, in the context of the present invention a range of values indicated for a certain parameter, for example the weight or the volume of a component in a mixture, includes the upper and the lower limits of the range, e.g. if the content in weight, or in volume, of a component A in a mixture is indicated as "X to Y", the content of A can be X, Y or any of the intermediate values.

Unless otherwise indicated, the data relative to the peaks in the XRPD pattern are meant within the common uncertainty due to the instrument measurement, typically $\pm$ 0.2 degrees 2$\theta$, when collected with the K$\alpha$ radiation of copper ($\lambda$ =1.5419 Å) at room temperature. The present invention provides, in one aspect, a new form of N-[(2S)-1-[3-(3-chloro-4-cyanophenyl)-1H-pyrazol-1-yl]-propan-2-yl]-5-(1-hydroxyethyl)-1H-pyrazole-3-carboxamide (darolutamide), herein called nanocrystalline form or NC form or Form NC, said nanocrystalline form being characterized by having specific surface area (SSA) in the range from about 40 $m^2$/g to about 150 $m^2$/g, preferably from about 50 $m^2$/g to about 135 $m^2$/g, more preferably from about 60 $m^2$/g to about 135 $m^2$/g analyzed using the multi-point nitrogen adsorption technique based on the Brunauer, Emmett and Teller (BET) theory, for example using TriStar (II) automated gas adsorption analyzer, (Micromeritics, Inc.). The samples are suitably vacuum dried overnight at 45°C. The volumetric method can be used at the relative pressure range of 0.05 - 0.3 $p/p_0$ as reported in the experimental part. Specific surface area is an important physical property for an active pharmaceutical ingredient. This parameter can greatly influence the process of preparation of drugs, such as filtration, purification, blending, tableting as well as the characteristics of the active pharmaceutical ingredient especially its dissolution rate and, in turn, its bio-availability as generally known in the art, e.g. from Kyung Rok Chu et al. Effect of particle size on the dissolution behaviours of poorly water-soluble drugs, Arch. Pharm. Res., 2012 July, 35(7), 1187-95, DOI 10.1007/s12272-012-0709-3.

The results, described in the example 3, demonstrate that nanocrystalline form (Form NC) of the present invention exhibits higher SSA values if compared to those obtained from samples of Form I and of crystalline rounded

particles, synthetized respectively following the teaching of the patent applications WO 2016/120530 and WO 2018/162793, as reported in comparative examples 1a and 2.

In addition, the SSA values, calculated for the samples of Form I and of crystalline rounded particles, obtained respectively from comparative example 1a and 2, are consistent to that disclosed in WO 2018/162793 patent application and with those reported in annex 1, written by the applicant of said application during the prosecution of the European phase. As reported in comparative examples 1b and 1c, Form I prepared according to WO 2016/120530 was also ground both according to the teaching of EP 3 495 352 A1 (manually in a mortar for 5 minutes) and by an instrumental technique (micronization by FPS Q-Mill equipment): the obtained samples showed the same polymorphic Form I but with a lower degree of crystallinity (thus possibly originating potential stability issues) while SSA could be increased only very slightly with respect to the starting material, remaining well below the values characteristic of the Form NC of the present invention.

This shows that Darolutamide with a higher surface area cannot be obtained by simple common techniques from the previously known Form I, while the Form NC of the present invention intrinsically possesses an advantageously higher SSA by its nature.

In another aspect of the present invention, said nanocrystalline form possesses increased solubility in physiologically relevant aqueous media compared to Form I and to crystalline rounded particles. The solubility trials were performed dissolving all samples in aqueous media at both pH 1.0 and pH 7.5 at 37°C under atmospheric pressure and determining the concentration of saturated darolutamide solution under stirring, in the presence of undissolved material, by HPLC assay versus external reference standard. The results show that after 60 minutes Form I and crystalline rounded particles present the same solubility at both pH values, whereas nanocrystalline form of the present invention possesses a solubility eight times higher. Furthermore, Form I and crystalline rounded particles show the equilibrium solubility concentration of 0.02 mg/mL after 5 minutes at both pH conditions and this concentration value remains unchanged at 10 and 60 minutes timepoints, while the solubility of the nanocrystalline form of the present invention is in the range 0.12-0.16 mg/mL after 5 minutes. These results are crucial especially for BCS class II substances, as darolutamide, because for these drugs the bioavailability may be enhanced by increasing the solubility and the dissolution rate of the drug in the gastro-intestinal fluids. It is well known that one of the rate limiting step of this class of drugs is the solubility in the gastric fluid and not the absorption. Therefore, increasing the solubility increases the bioavailability and consequently, the daily dose can be significantly reduced as indeed the adverse outcomes of the patients.

In a specific aspect, said nanocrystalline form of darolutamide is further characterized by having a powder X-ray diffractogram comprising broad peaks at 10.2, 14.4, and 17.8 $\pm$ 0.2 degrees 2-Theta, when measured at room temperature with Cu-K$\alpha$ radiation having a wavelength of 1.5419 Å.

Preferably, said crystalline form of darolutamide is characterized by having a powder X-ray diffractogram additionally comprising each of the broad peaks at of 8.6 and 23.6 $\pm$ 0.2 degrees 2-Theta, when measured at room temperature with Cu-K$\alpha$ radiation having a wavelength of 1.5419 Å.

In another specific aspect said crystalline form of darolutamide is characterized by having a powder X-ray diffractogram comprising at least one of the following broad peaks at 6.2, 16.3, and 22.2 $\pm$ 0.2 degrees 2-Theta, when measured at room temperature with Cu-K$\alpha$ radiation having a wavelength of 1.5419 Å.

In another specific aspect, said nanocrystalline form of darolutamide is characterized by X-ray diffraction pattern shown in Figure 1.

As a non-limiting example, the list of peak positions related to the representative XRPD pattern of said nanocrystalline form is provided hereunder in Table 1. The peaks in Table 1 XRPD pattern are meant within the common uncertainty due to the instrument measurement $\pm$0.2 degrees 2$\theta$, when collected with the K$\alpha$ radiation of copper ($\lambda$ =1.5419 Å) at room temperature.

Table 1

| Peak number | 2theta [°] |
|---|---|
| 1 | 6.2 |
| 2 | 8.6 |
| 3 | 10.2 |
| 4 | 14.4 |
| 5 | 16.3 |
| 6 | 17.8 |
| 7 | 22.2 |
| 8 | 23.6 |

In a further specific aspect said nanocrystalline form of darolutamide is characterized by having DSC profile, when measured with DSC at a heating rate of 10°C/min, that shows a broad endothermic transition with a peak temperature enclosed in the range from about 130 to about 150°C. Such transition relates to the melting of the nanocrystalline form of the present invention. In another specific aspect darolutamide is characterized by having DSC profile, when measured with DSC at a heating rate of 10°C/min, that shows a broad endothermic transition with a peak temperature enclosed in the range from about 130 to about 150°C, followed by an exothermic transition with a peak temperature enclosed in the range from about 140 to about 155°C, followed by a further endothermic transition having peak temperatures enclosed in the range from about 160 to about 180°C, preferably in the range from about 164 to about 176°C. In another specific aspect the first and the second thermal events may be only partially resolved.

In further specific aspect said nanocrystalline form of darolutamide is characterized by having DSC thermogram shown in Figure 2.

In yet further specific aspect these thermal events may show relative intensity modification if compared each other.

In another specific aspect said nanocrystalline form of darolutamide is characterized by having a $^{13}C$ CPMAS NMR spectrum comprising peaks at 163.3 ± 0.2 ppm, 107.3 ± 0.2 ppm 102.4 ± 0.2 ppm and 57.4 ± 0.2 ppm.

In another specific aspect said nanocrystalline form of darolutamide is characterized by having a $^{13}C$ CPMAS NMR spectrum comprising additionally peaks at 19.5 ± 0.2 ppm, 47.4 ± 0.2 ppm, 62.2 ± 0.2 ppm, 104.8 ± 0.2 ppm, 111.1 ± 0.2 ppm, 115.0 ± 0.2 ppm, 122.5 ± 0.2 ppm, 126.2 ± 0.2 ppm, 134.1 ± 0.2 ppm, 139.2 ± 0.2 ppm, 148.0 ± 0.2 ppm, 152.1 ± 0.2 ppm, 162.4 ± 0.2 ppm.

In yet further specific aspect, said nanocrystalline form of darolutamide is characterized by $^{13}C$ CPMAS NMR spectrum shown in Figure 3.

In another specific aspect said nanocrystalline form of darolutamide is characterized by having an infrared spectrum shown in Figure 4.

Still in another specific aspect the nanocrystalline form according to the present invention has a crystallite size, calculated by Scherrer equation, in a range from about 5 nm to about 11 nm, preferably from about 6 nm to about 8 nm.

Scherrer equation was applied to the most isolated and clearly defined peaks in the X-ray patterns of said nanocrystalline form, using a profile fitting (PF) technique with a "split pseudo-Voigt" description for the slightly asymmetric peak shapes to better determine the experimentally measurable peak widths values, necessary to calculate the crystallite size as reported in detail in the experimental part. The estimated crystallite size obtained via said mathematical method agrees with the size of the microstructure observed using the scanning electron microscope (SEM). According with SEM image shows in Figure 5, it is possible to argument that magnifying at 1 μm, the sample presents a complex framework composed by submicrometer-sized very thin filaments which, in turn, are formed by other substructures, with much smaller size, well below the 30 nm regime. No morphologically crystalline particles can be observed down to this 30 nm limit, indicating that, in line with the XRPD results, the material is definitely nanosized.

In another aspect the crystalline form according to the present invention is chemically and physically stable: it does not change its polymorphic form assessed by X-Ray Powder Diffraction (XRPD) and its chemical purity measured by high performance liquid chromatography (HPLC) analysis, does not decrease over storage at 25°C and 40°C / 75% RH for several months, as reported in the experimental part.

In another aspect, the present invention relates to a process for the preparation of darolutamide in nanocrystalline form comprising the steps of:

(i) providing a solution of darolutamide in an organic solvent selected from the group consisting of 2-methyltetrahydrofuran, tetrahydrofuran, dioxane, and any mixture thereof, said solution containing an amount of water between about 2 % to about 20% by weight of the total solution;
(ii) optionally filtering;
(iii) cooling the obtained solution to -5/15°C;
(iv) optionally seeding before or during the antisolvent addition described in (v);
(v) adding an antisolvent selected from C5-C7 linear or branched alkane, cycloalkane, substituted cycloalkane, toluene, symmetric ethers, and asymmetric ethers, characterized in that the temperature of the mixture is maintained in the range of from -5 to 15°C during the addition;
(vi) optionally adding other portions of the antisolvent used in step (v) to cause the complete precipitation of the solid;
(vii) recovering the resulting solid and, optionally, drying it.

Darolutamide used in the above described process could be prepared according one of the teaching disclosed in the prior art or preferably from the reduction of ketodarolutamide Formula (II), following the procedures reported in the experimental part (examples 12 a-e). Ketodarolutamide could be synthetized according one of the teaching disclosed in the prior art, especially following the procedure reported in example 34 of WO 2011/051540.

In another specific aspect step (i) of the above described process is performed using a solution provided by

dissolving darolutamide in any solid forms, in an organic solvent selected from the group consisting of 2-methyltetrahydrofuran, tetrahydrofuran, dioxane, and any mixture thereof, containing an amount of water between about 2 % to about 20 % by weight of the total solution. In step (i) the amount of water content by weight of the total solution could be measured according to Karl Fischer method. The water content % by weight relates to the water present when all the components comprising the mixture are mixed together, i.e. solvent, water, and darolutamide.

Optionally, if two phases are formed after step (i), a further step (i') could be performed, said step (i') comprising separating the phases and, if present, discarding the aqueous phase. The separation might be performed as explained below and included in optional step (ix), by decanting the phases and discarding the aqueous phase.

In yet another specific aspect the provided solution of step (i) is maintained in a range of temperature from about 25 to about 70°C, more preferably from about 45 to about 60°C for about 30 minutes to dissolve completely the solid. The exact temperature needed for dissolution also depends on the applied darolutamide concentration.

In a further specific aspect, the darolutamide concentration of the solution in step (i) usually ranges from about 160 to about 30 g/L, preferably from about 145 to about 45 g/L, and more preferably from about 65 to about 50 g/L. Most preferably the darolutamide concentration of the solution in step (i) is from about 65 g/L to about 45 g/L.

More preferably step (i) is performed providing a solution of 2-methyltetrahydrofuran, containing an amount of water between about 2 % to about 20 % by weight of the total solution, as could be measured according to Karl Fischer method, having a darolutamide concentration of about 65 to about 50 g/L at the temperature in a range from about 45 to about 50°C.

Optionally the solution may be filtered (optional step (ii)) at the same temperature of the solution provided in step (i), in order to obtain a clear solution, removing any potentially not dissolved particles of darolutamide. Optionally, step (i') could be performed after optional step (ii) of filtering.

The solution is cooled to -5/15°C (step iii) and optionally seeded (step iv) in order to promote crystallization and to control the particle size of the obtained solid.

The seeding could be performed using a seed crystal amount in a range from about 1 to about 10 % by weight, based on the total amount of darolutamide present in the solution. The seed crystal is usually in the nanocrystalline form of the present invention.

The seeding process (iv) may be carried out before or during the antisolvent addition of step (v).

The nanocrystalline form of darolutamide starts to precipitate by adding antisolvent, selected from $C_5$-$C_7$ linear or branched alkane, cycloalkane, substituted cycloalkane, toluene, symmetric ether, and asymmetric ethers, to the solution obtained in step (iii), optionally seeded, and by maintaining the temperature of the mixture in the range of from about -5 to about 15°C during the whole addition.

Preferably the antisolvent is added portion-wise to maintain the right temperature of the process. More preferably a first portion of antisolvent is added, the solution is maintained under stirring for about 0.5 to about 3 hours, then a second portion and a third one may be added to complete the precipitation.

The applied antisolvent-solvent volume ratio usually ranges from about 1:1 to about 3:1, preferably 2:1, wherein the solvent ratio is based on the organic solvent volume present in the solution provided in step (i).

The antisolvent is selected from $C_5$-$C_7$ linear or branched alkane, cycloalkane, substituted cycloalkane, toluene, symmetric ethers, and asymmetric ethers, preferably the antisolvent is selected from heptane and toluene, more preferably the antisolvent is heptane.

The mixture obtained from step (v) is preferably further stirred at a temperature from about -5 to about 15°C for a period ranging from about 1 to about 24, preferably from about 3 to about 16 hours in order to obtain darolutamide in nanocrystalline form in high yield. The obtained nanocrystalline form of darolutamide is then recovered from the mixture of step (v) and optionally dried.

Preferably the nanocrystalline form is separated from the mixture of step (v) by using any conventional method such as filtration, centrifugation or decantation, more preferably by filtration or centrifugation, advantageously by filtration.

In a further step the obtained solid may be washed with a suitable solvent, for example with the antisolvent used in step (v) for initiating the crystallization.

Optionally the obtained nanocrystalline form is dried using a conventional method for example the drying is performed at a temperature in the range of from about 20 to about 60 °C, preferably in the range of from about 30 to about 45°C under vacuum. Typically, drying is performed for a period in the range of from about 6 to about 72 hours, preferably from about 10 to about 48 hours, more preferably from about 12 to about 24 hours.

In another aspect of the present invention the solution of step (i) is obtained following the the steps comprising:

(viii) providing darolutamide dissolved in a mixture of an organic solvent selected from the group consisting of 2-methyltetrahydrofuran, tetrahydrofuran, dioxane, and any mixture thereof, and water, wherein the water amount is up to 20 % by weight of the total mixture, e.g. measured according to Karl Fischer method;

(ix) if two phases are present, decanting the phases and discharging the aqueous layer;

(x) optionally distilling the organic solvent at ambient pressure until a residual solution having a darolutamide

concentration from about 160 to about 30 g/L is obtained;

(xi) optionally adding another portion of fresh organic solvent or mixture thereof used in step (viii), and performing step x, in order to obtain a solution containing an amount of water between 2 % to 20 % by weight of the total solution, e.g. measured according to Karl Fischer method.

In a specific aspect the mixture provided in step (viii) is maintained in a range of temperature from about 25 to about 70°C, more preferably from about 45 to about 60°C for about 30 minutes in order to have darolutamide completely dissolved.

In another specific aspect the mixture provided in step (viii) of the above described process is performed dissolving darolutamide in any solid forms, in a mixture of an organic solvent selected from the group consisting of 2-methyltetrahydrofuran, tetrahydrofuran, dioxane, and any mixture thereof, and water, wherein the water amount, calculated as a percentage, is up to 20 % by weight of the total mixture. The amount of water can be measured, for example, according to Karl Fischer method.

Optionally the darolutamide mixture of step (viii) is obtained directly from the organic layer arising from the work-up of reactions that lead to obtain darolutamide as a product.

Preferably the darolutamide mixture of step (viii) is provided directly from the organic layer arising from the work-up of reduction of ketodarolutamide of Formula (II), wherein this reaction is carried out in 2-methyltetrahydrofuran, tetrahydrofuran, dioxane, or a mixture thereof, and water, in the presence of a hydride as reductive agent.

More preferably the reduction reaction is carried out in 2-methyltetrahydrofuran or dioxane, and water, using sodium borohydride as reductive agent.

Optionally sodium borohydride is added portion wise to the solution containing darolutamide at room temperature.

The reaction mixture is stirred for about 3-10 hours until complete conversion being obtained, maintaining the temperature in a range from about 20 to about 50°C, preferably at room temperature. Optionally the work-up of the reaction is performed adding a solution of HCl 1N and stirring the mixture for about 15 to about 30 minutes at room temperature. Then the aqueous phase is discarded and an amount of water up to 20 % by weight of the total the organic phase is added to provide the solution to be used directly in step (viii).

The obtained solution is then decanted, and the aqueous layer is discharged, maintaining the temperature during all the operation of step (ix) in a range from about 25 to about 70°C, more preferably from about 45 to about 60°C for about 30 minutes, in order to have darolutamide completely dissolved.

The obtained organic layer is optionally distilled in step (x) at ambient pressure until obtaining a residual solution having a darolutamide concentration from 160 to 30 g/L, preferably from 145 to about 45 g/L, more preferably from about 65 to about 50 g/L.

Optionally another portion of fresh organic solvent or mixture thereof, selected from the solvents used in step (viii), is added and step (x) is performed again in order to obtain a solution having a darolutamide concentration from 160 to 30 g/L, preferably from 145 to about 45 g/L, more preferably from about 65 to about 50 g/L, said solution containing an amount of water between about 2 % to about 20 % by weight of the total solution, e.g. measured according to Karl Fischer method. The quantity of fresh organic solvent or mixture thereof, selected from the solvents used in step (viii), is added in the range from about 1:10 to about 4:10, calculated in volume ratio referred to the total volume of the same solvent or mixture thereof used in step (viii). In another specific aspect, the volume ratio of fresh organic solvent or mixture thereof, selected from the solvents used in step (viii), includes any two of the following values 1.3:10, 2:10, 3:10.

In another aspect the present invention provides a pharmaceutical formulation comprising nanocrystalline form of darolutamide and, optionally, at least one excipient.

Said formulation can be suitably prepared, for example, by mixing nanocrystalline form of darolutamide in an amount that ranges from about 25 % to about or 75 %, preferably from about 35 % to about 55 %, based on the total weight of the pharmaceutical composition, with one or more pharmaceutically acceptable excipients conventionally used for formulations.

In another aspect nanocrystalline form of the present invention can be formulated as a tablet comprising at least one pharmaceutically acceptable excipient suitable for their preparation and nanocrystalline form of darolutamide, wherein the amount of said nanocrystalline form is in the range from about 25 % to about 75 %, preferably from about 35 % to about 55 %, based on the total weight of the tablet.

The tablets may be prepared using the conventional method known in the art.

A suitable method is the wet granulation technique, as reported as non-limiting example in the experimental part. Said formulation can be suitably prepared first by mixing nanocrystalline form of darolutamide with the required excipients, preferably with at least one filler, *e.g* with lactose monohydrate anhydrous, calcium hydrogen phosphate and a mixture thereof, at least one binder, *e.g* with povidone, and a first portion of disintegrant.

Said disintegrant is added in a portion between 0.2 % to 0.8 % by weight, preferably the disintegrant is croscarmellose sodium. The mixture is then granulated in a suitable granulator vessel, for example wet high shear

granulator, using purified water as granulating liquid. The wet granules can be dried, preferably in a fluid bed dryer, then the rest of the disintegrant can be added to the granules. Lubricant, *e.g* magnesium stearate, is then added to the mass of the previous step followed by blending. The granulated mass is then compressed to obtain a tablet.

In yet another aspect the present invention provides a pharmaceutical formulation comprising a homogeneous solid dispersion of darolutamide obtained by milling said nanocrystalline form using conventional milling technology such as fluid jet mill. Said milling technology may produce finer pharmaceutical powders, where the final particle size is in the range required to support the preparation of a mixture for tablet formulation.

In yet another specific aspect the present invention provides a film-coated tablet to facilitate tablet swallowing, comprising the nanocrystalline form of darolutamide and, optionally, at least one excipient.

The following non-limiting examples are illustrative for the disclosure and are not to be construed as to be in any way limiting for the scope of the invention.

## EXPERIMENTAL PART

<u>Example 1a (Comparative): Preparation of darolutamide Form I</u>

(Repetition of example 1 of WO 2016/120530)

40.0 g of darolutamide, water (30 mL) and acetonitrile (570 mL) were charged in a 1000 mL glass reactor and the resulting mixture was dissolved by heating at 78°C. The solution was cooled under stirring to room temperature and stirred at the same temperature for 3 days. The obtained suspension was filtered, and the solid cake was washed with 160 mL of acetonitrile:water (95:5) mixture. The wet solid was dried under vacuum at 40°C for 12 hours then at 60°C for 2 hours, yielding 38.4 g (y= 90.6 %) of darolutamide. The XRPD trace of the resulting sample is reported in Fig. 13.

<u>Example 1b (Comparative): Manual grinding of Darolutamide Form I</u>

(Repetition of Example 13 of EP 3 495 352 A1)

About 50 mg of Darolutamide Form I prepared according to WO 2016/120530 were manually ground in a mortar for 5 minutes; the resulting solid was analysed by XRPD (XRPD trace reported in Fig. 13).

The procedure was replicated on 4 g to have sufficient material for SSA determination of the resulting solid (data reported in Table 2 of Example 13).

<u>Example 1c (Comparative): Instrumental grinding of Darolutamide Form I</u>

(Similar to Example 13 of EP 3 495 352 A1, but by means of a milling equipment)

About 10 g of Darolutamide Form I prepared according to WO 2016/120530 were milled in an FPS Q-Mill 2 equipment using the following instrumental parameters:

| Process gas | Process gas Temp. | Dosing | Injection line pressure | Milling line pressure | Milling nozzles diameter | Injection nozzle diameter | Trial duration |
|---|---|---|---|---|---|---|---|
| Nitrogen | 20-25°C | 0.5 g/h | 6 barg | 5 barg | 0.4 mm | 1.2 mm | 20 min |

The milled solid was analysed by XRPD (XRPD trace reported in Fig. 13) and for SSA determination (data reported in Table 2 of Example 13).

<u>Example 2 (Comparative): Preparation of crystalline rounded particles</u>

(Repetition of example 3 of WO 2018/162793)

15.0 g of darolutamide, water (100 mL) and ethanol (110 mL) were charged in a 500 mL glass reactor and the resulting mixture was dissolved by heating to 75°C. 786 mg of activated carbon SX Ultra and 1.5 g of celite were added, followed by stirring for 1 h. The suspension was then filtered as hot and the filtrate was transferred into a 500 mL glass reactor. Carbon/celite cake was washed with 15.5 mL of ethanol. The resulting washing liquid was added to the solution in

the glass reactor. Then the solution was cooled to 70°C in 10 minutes and to 60°C in 10 minutes. Then the solution was cooled to 30°C and 75 mL of water were added simultaneously. The resulting suspension was stirred further for 30 minutes, the precipitate was filtered and the obtained solid cake was washed with water. The wet solid was dried under vacuum at 70°C, yielding 12.38 g (y= 82.5%) of darolutamide.

Example 3: Preparation of nanocrystalline form of darolutamide starting from darolutamide in a solid form

100 g of darolutamide were suspended in 2-methyltetrahydrofuran (1000 mL) and water (200 mL), and the mixture was heated to 45-50°C until complete dissolution of solid was observed. Resulting clear phases were separated: bottom aqueous phase was discharged and the organic layer was distilled at ambient pressure until a residual volume of about 700 mL was achieved. To the solution was added 2-methyltetrahydrofuran (300 mL), then the mixture was distilled again at ambient pressure until a residual volume of about 700 mL was reached. Resulting solution with a water content of about 2.6 % measured by Karl Fischer analysis was filtered and cooled to -5/ 5°C, then heptane (200 mL) was added dropwise in about 15 minutes. The mixture was maintained under stirring at -5/ 5°C for at least 3 hours. To the resulting suspension heptane (1200 mL) was added dropwise in about 30 minutes and the mixture was maintained under stirring at -5/ 5°C for at least 16 hours. The suspension was filtered and the solid was washed with heptane (300 mL). Wet product was dried under vacuum at 50°C for 17-20 hours until constant weight. About 90 g (y= 90.0 %) of nanocrystalline form of darolutamide as off-white powder were obtained.

Example 4: Preparation of nanocrystalline form of darolutamide starting from darolutamide in a solid form

100 g of darolutamide were suspended in 2-methyltetrahydrofuran (1000 mL) and water (200 mL), and the mixture was heated to 45-50°C until complete dissolution of solid was observed. Resulting clear phases were separated: bottom aqueous phase was discharged, and the organic layer was distilled at ambient pressure until a residual volume of about 700 ml was achieved. To the solution was added 2-methyltetrahydrofuran (200 mL), then the mixture was distilled again at ambient pressure until a residual volume of about 700 mL was reached. Resulting solution with a water content of about 3.6 % measured by Karl Fischer analysis was cooled to -5/ 5°C and a seed of nanocrystalline form of darolutamide (1 % w/w) was added. Resulting suspension was maintained under stirring at this temperature for 15-20 minutes and then heptane (200 mL) was added dropwise in about 30 minutes. The mixture was maintained under stirring at -5/ 5°C for at least 3 hours. To the resulting suspension heptane (1200 mL) was added dropwise in about 30 minutes and the mixture was maintained under stirring at -5/ 5°C for at least 16 hours. The suspension was filtered and the solid was washed with heptane (300 mL). Wet product was dried under vacuum at 50°C for 17-20 hours until constant weight. About 92 g (y= 92.0 %) of nanocrystalline form of darolutamide as off-white powder were obtained.

Example 5: Preparation of nanocrystalline form of darolutamide starting from darolutamide in a solid form

50 g of darolutamide were suspended in 2-methyltetrahydrofuran (500 mL) and water (100 mL), and the mixture was heated to 45-50°C until complete dissolution of solid was observed. Resulting clear phases were separated: bottom aqueous phase was discharged and the organic layer was distilled at ambient pressure until a residual volume of about 350 mL was achieved. Resulting solution with a water content of about 5.7 %, measured by Karl Fischer analysis, was cooled to -5/ 5°C, then heptane (200 mL) was added dropwise in about 30 minutes. The mixture was maintained under stirring at -5/ 5°C for at least 3 hours. To the resulting suspension heptane (250 mL) was added dropwise in about 30 minutes and the mixture was maintained under stirring at -5/ 5°C for at least 1 hour. Then another portion of heptane (250 ml) was added in about 30 minutes and the mixture was maintained under stirring at -5/ 5°C for at least 16 hours. The suspension was filtered and the solid was washed with heptane (150 mL). Wet product was dried under vacuum at 50°C for 17-20 hours until constant weight. About 45 g (y= 90%) of nanocrystalline form of darolutamide as off-white powder were obtained.

Example 6: Preparation of nanocrystalline form of darolutamide starting from darolutamide in a solid form

3 g of darolutamide_were suspended in 2-methyltetrahydrofuran (30 mL) and water (6 mL), and the mixture was heated to 45-50°C until complete dissolution of solid was observed. Resulting clear phases were separated: bottom aqueous phase was discharged and the organic layer was distilled at ambient pressure until a residual volume of about 22 mL was achieved. Resulting solution with a water content of about 5.8 % measured by Karl Fischer analysis was filtered. The filtrate was cooled to 0-10°C and then heptane (42 mL) was added dropwise in about 60 minutes. The mixture was maintained under stirring at 0-10°C for at least 16 hours. The suspension was filtered and the solid was washed with heptane (10 mL). Wet product was dried under vacuum at 50°C for 17-20 hours until constant weight. About 2.5 g (y= 83.3 %) of nanocrystalline form of darolutamide as off-white powder were obtained.

Example 7: Preparation of nanocrystalline form of darolutamide starting from darolutamide in a solid form

30 g of darolutamide were suspended in 2-methyltetrahydrofuran (450 mL) and water (60 mL), and the mixture was heated to 45-50°C until complete dissolution of solid was observed. Resulting clear phases were separated: bottom aqueous phase was discharged and the organic layer was distilled at ambient pressure until a residual volume of about 300 mL was achieved. To the solution was added 2-methyltetrahydrofuran (60 mL), then the mixture was distilled again at ambient pressure until a residual volume of about 300 mL was reached. The resulting solution with a water content of about 3.7%, measured by Karl Fischer analysis, was filtered than cooled to 0-10°C. Heptane (600 mL) was added dropwise in about 60 minutes. The mixture was maintained under stirring at 0-10°C for at least 16 hours. The suspension was filtered and the solid was washed with heptane (90 mL). Wet product was dried under vacuum at 50°C for 17-20 hours until constant weight. About 29 (y= 96.7 %) g of nanocrystalline form of darolutamide as off-white powder were obtained.

Example 8: Preparation of nanocrystalline form of darolutamide starting from darolutamide in a solid form

40 g of darolutamide were suspended in 2-methyltetrahydrofuran (400 mL) and water was added until to obtain an amount of water of about 4.3% by weight of the total solution, measured according to Karl Fischer method. The mixture was heated to 45-50°C until complete dissolution of solid was observed, then was filtered and cooled to 0-10°C. A seed of nanocrystalline form of darolutamide (2 % w/w) was added, then 800 mL of heptane were added dropwise in about 60 minutes. The mixture was maintained under stirring at 0-10°C for at least 20 hours. The suspension was filtered and the solid was washed with heptane (100 mL). Wet product was dried under vacuum at 50°C for 17-20 hours until constant weight. About 35 g (y= 87.5%) of nanocrystalline form of darolutamide as off-white powder were obtained.

Example 9: Preparation of nanocrystalline form of darolutamide starting from darolutamide in a solid form

20 g of darolutamide were suspended in 2-methyltetrahydrofuran (200 mL) and water (40 mL), and the mixture was heated to 45-50°C until complete dissolution of solid was observed. Resulting clear phases were separated: bottom aqueous phase was discharged. To the solution was added 2-methyltetrahydrofuran (40 mL), then the mixture was distilled at ambient pressure until a residual volume of about 140 mL was reached. To the solution was added 2-methyltetrahydrofuran (40 mL), then the mixture was distilled at ambient pressure until a residual volume of about 140 mL was reached. Resulting solution with a water content of about 3.4 % measured by Karl Fischer analysis was cooled to 0-5°C and a seed of nanocrystalline form of darolutamide (1 %w/w) was added. Resulting suspension was maintained under stirring at this temperature for 15-20 minutes and then toluene (40 mL) was added dropwise in about 30 minutes. The mixture was maintained under stirring at 0-5°C for about 3 hours. To the resulting suspension toluene (240 mL) was added dropwise in about 30 minutes and the mixture was maintained under stirring at 0-5°C for about 16 hours. The suspension was filtered and the solid was washed with toluene (60 mL). Wet product was dried under vacuum at 50°C for 17-20 h until constant weight. About 18 g (y= 90.0 %) of nanocrystalline form of darolutamide as off-white powder were obtained.

Example 10: Preparation of nanocrystalline form of darolutamide starting from a darolutamide solution obtained directly from the reduction of 3-acetyl-N-[(2S)-1-[3-(3-chloro-4-cyanophenyl)pyrazol-1-yl]propan-2-yl]-1H-pyrazole-5-carboxamide (Ketodarolutamide)

In a glass reactor 20 g (50.4 mmol) of Ketodarolutamide, 300 mL of 2-methyltetrahydrofuran, 40 mL of water were added. $NaBH_4$ (1.42 g, 37.5 mmol) was added in one portion at room temperature and the mixture was stirred at the same temperature for 17 hours. 1N HCl was added up to pH about 4, the mixture was stirred for about 15 minutes, then the aqueous phase was discarded and the organic phase was washed two times at 55-60°C with 100 mL of $H_2O$.

The organic phase was distilled at atmospheric pressure until 200 mL of solution were obtained. To the solution were added 40 mL of 2-methyltetrahydrofuran and the solvent was distilled again to obtain 140 mL of residue with a water content of about 2.5 % measured by KF analysis. Then the solution was cooled to 0-5°C and 40 mL of heptane were added in about 15-20 minutes. The suspension was stirred for 3 hours at 0-5°C, then additional heptane (240 mL) was added in about 30 minutes to complete the precipitation. The suspension was stirred for 14 hours maintaining the temperature at 0-5°C, then solid was recovered by filtration. The solid cake was washed with 80 mL of heptane and the wet solid was dried in a vacuum oven at 50°C for about 16 hours.

Example 11: Preparation of nanocrystalline form of darolutamide starting from a darolutamide solution obtained directly from the reduction of 3-acetyl-N-[(2S)-1-[3-(3-chloro-4-cyanophenyl)pyrazol-1-yl]propan-2-yl]-1H-pyrazole-5-carboxamide (Ketodarolutamide)

In a glass reactor 50 g (126.0 mmol) of Ketodarolutamide, 750 mL of 2-methyltetrahydrofuran, 100 mL of water

were added. $NaBH_4$ (3.60 g, 95.2 mmol) was added in one portion at room temperature and the mixture was stirred at the same temperature for 17 hours. 1N HCl was added up to pH about 4, the mixture was stirred for about 20 minutes, then the aqueous phase was discarded, and the organic phase was washed two times at 55-60°C with 250 mL of $H_2O$.

The organic phase was distilled at atmospheric pressure until 350 mL of solution with a water content of about 5.9 % measured by KF analysis, were obtained. Then the solution was cooled to 0-5°C and 700 mL of heptane were added in about 3-4 hours. The suspension was stirred for 14 hours maintaining the temperature at 0-5°C, then the solid was recovered by filtration. The solid cake was washed with 200 mL of heptane and the wet solid was dried in a vacuum oven at 50°C for about 16 hours.

Example 12: Reduction 3-acetyl-N-[(2S)-1-[3-(3-chloro-4-cyanophenyl)pyrazol-1-yl]propan-2-yl]-1H-pyrazole-5-carboxamide (Ketodarolutamide) performed in different solvents

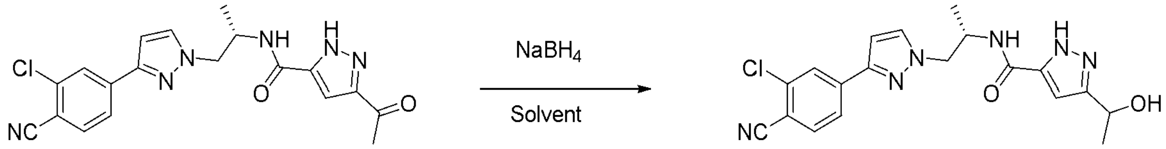


II I

12 a: Reduction performed in 2-methyltetrahydrofuran

8.93 g (22.5 mmol) of Ketodarolutamide, 134 mL of 2-methyltetrahydrofuran and 18 mL of $H_2O$ were charged in a 250 mL round bottom flask equipped with mechanical stirrer under a nitrogen atmosphere. To the resulting suspension $NaBH_4$ (0.638 g, 16.85 mmol) was added in three portions at room temperature. The mixture was stirred for 5 hours. 1N HCl was added up to pH about 4, the mixture was stirred for about 15 minutes at room temperature, then the aqueous phase was discarded and the organic phase was extracted two times at 55-60°C with 20 mL of $H_2O$. The resulting organic phase was evaporated at 40-45°C under vacuum yielding 8.2 g of darolutamide as a white solid ($^1H$ NMR signals are reported at the end of the reduction procedures).

12 b: Reduction performed in 1,4-dioxane

500 mg (1.26 mmol) of Ketodarolutamide were suspended in a mixture of 1,4-dioxane (5 mL) and water (1 mL) under a nitrogen atmosphere. $NaBH_4$ (31 mg, 0.819 mmol) was added in one portion at room temperature and the mixture was stirred at the same temperature for 24 hours until complete conversion. 1N HCl was added up to pH about 1-2, the mixture was stirred for 1 hour and then neutralized with 1N NaOH. Dioxane was evaporated at 40-45°C under vacuum and the aqueous residue was extracted with 2-methyltetrahydrofuran (10 mL); the organic layer was washed with water (10 mL), dried over $Na_2SO_4$ and evaporated at 40-45°C under vacuum yielding 500 mg of darolutamide as a white solid ($^1H$ NMR signals are reported at the end of the reduction procedures).

12 c: Reduction performed in tetrahydrofuran

The reaction was performed according to the procedure of example 12 b replacing 1,4-dioxane with tetrahydrofuran as a solvent. The mixture was stirred at room temperature for 24 hours until complete conversion. 1N HCl was added up to pH about 1-2, the mixture was stirred for 1 hour at room temperature and then neutralized with 1N NaOH. Tetrahydrofuran was evaporated at 40-45°C under vacuum and the aqueous residue was extracted with 2-methyltetrahydrofuran (10 mL); the organic layer was washed with water (10 mL), dried over $Na_2SO_4$ and evaporated at 40-45°C under vacuum yielding 480 mg of darolutamide as a white solid ($^1H$ NMR signals are reported at the end of the reduction procedures).

12 d: Reduction performed in methyl tert-butyl ether

The reaction was performed according to the procedure of example 12 b replacing 1,4-dioxane with methyl tert-butyl ether as a solvent. After 20 hours the reaction mixture remained a suspension. Methyl tert-butyl ether (1.5 mL) and $NaBH_4$ (16 mg) were added again and stirring was continued for 14 hours. After 4 hours about 50% conversion was observed by HPLC analysis. 1N HCl was added up to pH about 1-2. After 1 hour the mixture was neutralized with 1N NaOH and filtered. The solid was purified using a preparative HPLC system to obtain 29 mg of darolutamide as a white solid ($^1H$

NMR signals are reported at the end of the reduction procedures).

12 e: Reduction performed in cyclopentyl methyl ether

The reaction was performed according to the procedure of example 12 b, replacing 1,4-dioxane with cyclopentyl methyl ether. After 20 hours cyclopentyl methyl ether was added and the mixture was heated at 45°C for 4 hours and then at 65°C for additional 4 hours. The solution was cooled to room temperature, then 16 mg of $NaBH_4$ were added. After 16 hours about 55% conversion was observed by HPLC analysis. 1N HCl was added up to pH about 1-2. After 1 hour the mixture was neutralized with 1N NaOH and filtered. The solid was purified using a preparative HPLC system to obtain 35 mg of darolutamide as a white solid having the $^1H$ NMR signals reported here below.

$^1H$ NMR of darolutamide (500 MHz, Methanol-d4) δ 8.05 (dd, J = 4.4, 1.5 Hz, 1H), 7.93 (d, J = 8.2 Hz, 1H), 7.81 (d, J = 8.1 Hz, 1H), 7.72 (d, J = 2.5 Hz, 1H), 6.78 (dd, J = 2.4, 0.7 Hz, 1H), 6.58 (s, 1H), 4.93 (q, J = 6.8 Hz, 1H), 4.57 (m, 1H), 4.38 (qd, J = 13.8, 6.0 Hz, 2H), 1.51 (d, J = 6.6 Hz, 3H), 1.26 (d, J = 6.8 Hz, 3H).

Example 13: Determination of specific surface area (SSA)

The specific surface area was determined for five batches of nanocrystalline Form of darolutamide prepared according to the present invention and for samples of Form I and crystalline rounded particles obtained respectively from comparative example 1 and 2 as a comparison.

The specific surface area was measured using the multi-point nitrogen adsorption technique based on the Brunauer, Emmett and Teller (BET) theory using TriStar (II) automated gas adsorption analyzer (Micromeritics, Inc.). All the samples were dried under vacuum overnight hours at 45 °C. The volumetric method was applied at the relative pressure range 0.05 -0.3 $p/p^0$, and the Specific Surface Area values were calculated in said pressure range using "Multi-Point BET" calculation method based on eleven linear points.

The-results are reported in Table 2.

Table 2

| Sample Example | Specific Surface Area (SSA) Multi-point BET ($m^2/g$) |
|---|---|
| Form I Comparative example 1a | 26.6 |
| Crystalline rounded particles Comparative example 2 | 11.6 |
| Manually ground Form I Comparative example 1b | 29.5 |
| Form I milled by Q-Mill Comparative example 1c | 34.4 |
| Form NC Example 3 | 134.0 |
| Form NC Example 4 | 111.7 |
| Form NC Example 5 | 66.8 |
| Form NC Example 10 | 121.6 |
| Form NC Example 11 | 66.6 |
| Form NC Example 23 | 46.0 |
| Form NC Example 24 | 43.8 |

The results shown that samples of nanocrystalline Form NC of darolutamide according to the present invention exhibit higher value of specific surface area if compared with that of samples respectively of Form I and of crystalline rounded particles, obtained in comparative examples 1a and 2, and with that of samples of ground or milled Form I obtained in comparative examples 1b and 1c (thus indicating that ground or milled Form I is a different solid form than nanocrystalline Form NC). Furthermore, the SSA values of samples respectively of Form I and of crystalline rounded particles obtained in comparative examples 1a and 2 are similar to that reported in WO 2018/162793 patent application and in the annex 1, written by the applicant during the prosecution in the European Phase of EP 3 592 732 (Table 3).

Table 3

| Crystalline rounded particles sample A unmilled | Crystalline rounded particles sample B unmilled | Form I |
|---|---|---|
| values reported in WO 2018/162793 | values reported in WO 2018/162793 | value reported by the applicant in annex 1 during the European patent prosecution of WO 2018/162793 |
| Specific Surface Area (SSA) Multi-point BET: 13 $m^2/g$ | Specific Surface Area (SSA) Multi-point BET: 12 $m^2/g$ | Specific Surface Area (SSA) Multi-point BET: 22.1 $m^2/g$ |

Example 14: Comparison of solubility of nanocrystalline Form, Form I and crystalline rounded particle

The solubility of nanocrystalline Form of darolutamide according to the present invention, of Form I and crystalline rounded particle obtained respectively from comparative example 1 and 2, were determined in aqueous media at selected pH as follows: buffer pH 1.0 (HCl 0.1 N) and buffer pH 7.5.

Under the aqueous media conditions above, solubility data should be obtained at 37°C and atmospheric pressure by determining the concentration of saturated darolutamide solution under stirring, in the presence of undissolved material, by HPLC assay versus external reference standard (i.e. Darolutamide).

HPLC method

Equipment: Waters Alliance e2695 chromatograph (Waters) equipped with 2998 PDA UV-Visible Detector.
Software: Empower 3 Chromatography Data System (Waters)
Column: XBridge Phenyl 3.5 um 4.6x150mm
Guard column: Gemini NX (Phenomenex) or equivalent
Column temperature: 40°C
Ammonium formate solution: Dissolve 15.765g of Ammonium formate in 250mL of Water to prepare a 1M solution of Ammonium formate (AF Stock)
Mobile Phase A: Dilute 10mL of AF Stock solution to 1000mL of Water. Adjust pH to 4.5.
Mobile Phase B: Methanol
Elution Mode: Gradient

| *Time (mins.)* | *A (%)* | *B (%)* |
|---|---|---|
| 0 | 45 | 55 |
| 10 | 45 | 55 |
| 13 | 5 | 95 |
| 15 | 5 | 95 |
| 16 | 45 | 55 |
| 25 | 45 | 55 |

Sample tray temperature: 25°C
Injection volume: 5 μL
UV detection: 280 nm
Analysis time: 15 minutes
Total Run time: 25 minutes
Under these conditions, Retention Time (RT) of darolutamide is typically 7.8 minutes

Reference Standard solution preparation:

Eight reference standard solutions having different concentrations were accurately prepared has reported here below.

Darolutamide Reference Standard Solution "CRS 0.25" (0.25 mg/mL of darolutamide)

To a 100 mL volumetric flask, transfer 25 mg accurately weighed of darolutamide reference standard (CRS) followed by about 80 mL of methanol, shake until complete dissolution and dilute to volume with MeOH.

Darolutamide Reference Standard Solution "CRS 0.20" (0.20 mg/mL of darolutamide)

To a 10 mL volumetric flask, transfer 6 mL of reference solution "CRS 0.25" and dilute to volume with methanol.

Darolutamide Reference Standard Solution "CRS 0.15" (0.15 mg/mL of darolutamide)

To a 25 mL volumetric flask, transfer 20 mL of reference solution "CRS 0.25" and dilute to volume with methanol.

Darolutamide Reference Standard Solution "CRS 0.125" (0.125 mg/mL of darolutamide)

To a 20 mL volumetric flask, transfer 10 mL of reference solution "CRS 0.25" and dilute to volume with methanol.

Darolutamide Reference Standard Solution "CRS 0.10" (0.10 mg/mL of darolutamide)

To a 25 mL volumetric flask, transfer 10 mL of reference solution "CRS 0.25" and dilute to volume with methanol.

Darolutamide Reference Standard Solution "CRS 0.075" (0.075 mg/mL of darolutamide)

To a 10 mL volumetric flask, transfer 3 mL of reference solution "CRS 0.25" and dilute to volume with methanol.

Darolutamide Reference Standard Solution "CRS 0.05" (0.05 mg/mL of darolutamide)

To a 50 mL volumetric flask, transfer 10 mL of reference solution "CRS 0.25" and dilute to volume with methanol.

Darolutamide Reference Standard Solution "CRS 0.01" (0.01 mg/mL of darolutamide)

To a 10 mL volumetric flask, transfer 1 mL of reference solution "CRS 0.10" and dilute to volume with methanol.

Calibration curve

HPLC method linearity of response was verified by injecting darolutamide reference solutions at known concentration in the range from 0.01 mg/mL to 0.25 mg/mL.

All levels are injected in triplicate but 100% who is injected six-times to verify RSD% at this level.

Linearity was evaluated by visual inspection of a plot of signals as a function of analyte concentration after which regression line by the method of least squares was calculated.

See the results in Table 4.

Table 4

| **Darolutamide injected reference solution** | | | **Area response** | | | | | | | |
|---|---|---|---|---|---|---|---|---|---|---|
| **% vs 0.10 mg/mL** | **RT (min)** | **Conc. (mg/mL)** | **A1** | **A2** | **A3** | **A4** | **A5** | **A6** | **Area avg** | **RSD %** |
| **0.01** | 7.79 | 0.01036 | 221260 | 220413 | 220672 | -- | -- | -- | **220782** | **0.20** |
| **0.05** | 7.80 | 0.05178 | 1109908 | 1119237 | 1098621 | -- | -- | -- | **1109255** | **0.93** |
| **0.075** | 7.80 | 0.07767 | 1655526 | 1654517 | 1656925 | -- | -- | -- | **1655656** | **0.07** |
| **0.10** | 7.80 | 0.10356 | 2204678 | 2202613 | 2205504 | 2222711 | 2196665 | 2195138 | **2204552** | **0.45** |
| **0.125** | 7.79 | 0.12945 | 2773085 | 2776019 | 2778325 | -- | -- | -- | **2775810** | **0.09** |
| **0.15** | 7.79 | 0.15534 | 3344002 | 3347763 | 3349614 | -- | -- | -- | **3347126** | **0.09** |

(continued)

| Darolutamide injected reference solution | | | Area response | | | | | | | |
|---|---|---|---|---|---|---|---|---|---|---|
| % vs 0.10 mg/mL | RT (min) | Conc. (mg/mL) | A1 | A2 | A3 | A4 | A5 | A6 | Area avg | RSD % |
| **0.20** | 7.78 | 0.20712 | 4448455 | 4459185 | 4454044 | -- | -- | -- | **4453895** | **0.12** |
| **0.25** | 7.77 | 0.25890 | 5543622 | 5542501 | 5544925 | -- | -- | -- | **5543683** | **0.02** |

The regression equation is: A= -7196 + 21480639*x
$R^2$: 0.9999

The coefficient of correlation r is greater than 0.990.

Zero figure is included within 95% CI interval of intercept for the regression line.

The linearity of the method is adequate in the considered inclusive concentration range 0.01 mg/mL - 0.25 mg/mL.

The calibration curve is reported in Figure 7.

Darolutamide test solution "T" for solubility determination

The samples of nanocrystalline form of darolutamide according to present invention, of Form I and of crystalline rounded particles were prepared by transferring 100 mg of darolutamide sample into a 40 mL test tube with screw cap, adding 20 mL of dissolution medium and placing the tube under magnetic stirring in a water bath at 37°C. After 5, 10 and 60 minutes an aliquot of the suspension was taken (about 1.5 mL) and filtered on 0.45 μm filter (syringe filter device -PTFE membrane filter). Then obtained clear solution was injected as is or diluted 0.8 to 1 mL with MeOH and injected in HPLC.

The dissolution media are prepared as follows:

- Buffer pH 7.5: dissolve 1.38 g of sodium dihydrogen phosphate ($NaH_2PO_4$) in 180 mL of water, adjust the pH to 7.5 with sodium hydroxide 4N and dilute to 1000 mL with water.
- Buffer pH 1.0: commercial solution of HCl 0.1 N

Calculation and Reporting:

Solution concentration is calculated by the following formula:

$$C(mg/mL) = \frac{A_T}{A_R} \times \frac{W_R}{D_R} \times \frac{1}{D_l} \times \frac{P}{100}$$

where:

C(mg/mL) is the concentration of darolutamide in diluent under investigation, expressed in mg/mL;
AT is the peak area of darolutamide in the test solution "T" chromatogram;
AR is the mean of peak area of darolutamide in the reference solution "CRS 0.10" chromatogram;
WR is the weight of darolutamide CRS 0.10 expressed as mg;
DR is the dilution to get "CRS 0.10" concentration of 0.10 mg/ mL starting from 25 mg nominal (250 mL);
P is the potency of darolutamide CRS (expressed in percentage).
DI is the dilution factor of darolutamide Test sample in HPLC vial calculated by the following formula:

$$D_l = \frac{V_T}{V_f}$$

where:

VT Is the volume of test solution to be diluted (*e.g.* 0.8 mL);
Vf is the final volume (*e.g.* 1 mL).

The solubility results are shown in the following table:

Table 5

| Dissolution condition of Darolutamide | Darolutamide Sample from Example | Crystalline form | Darolutamide Weight [mg] | Concentration of Darolutamide in saturated solution [mg/mL] | | | |
|---|---|---|---|---|---|---|---|
| | | | | Time point [minutes] | | | |
| | | | | 0 | 5 | 10 | 60 |
| **Buffer pH 1.0** | Example 1 (comparative) | Form I | 101.59 | 0.000 | 0.020 | 0.020 | 0.017 |
| | Example 2 (comparative) | Crystalline rounded particles | 100.47 | 0.000 | 0.023 | 0.022 | 0.022 |
| | Example 3 | Form NC | 100.53 | 0.000 | 0.159 | 0.162 | 0.117 |
| | Example 4 | Form NC | 102.30 | 0.000 | 0.137 | 0.146 | 0.113 |
| | Example 5 | Form NC | 101.69 | 0.000 | 0.117 | 0.126 | 0.113 |
| **Buffer pH 7.5** | Example 1 (comparative) | Form I | 102.04 | 0.000 | 0.020 | 0.021 | 0.019 |
| | Example 2 (comparative) | Crystalline rounded particles | 101.28 | 0.000 | 0.022 | 0.018 | 0.021 |
| | Example 3 | Form NC | 99.14 | 0.000 | 0.162 | 0.155 | 0.132 |
| | Example 4 | Form NC | 102.83 | 0.000 | 0.157 | 0.149 | 0.109 |
| | Example 5 | Form NC | 100.80 | 0.000 | 0.128 | 0.132 | 0.109 |

The time dependent solubility curves are reported in Fig 8.

Example 15: XRPD analysis

XRPD analysis was performed at room temperature by means of a theta/theta vertical scan Bruker AXS D8 Advance diffractometer. The instrument was equipped with a Cu-K$\alpha$ X-ray tube operating at 40 kV / 40 mA and generating radiation having $\lambda$= 1.5419 Å. The detector was a linear Lynxeye XE-T position sensitive set at 250 mm from the sample. Powder samples were deposited in the 12 mm x 0.2 mm hollow of the sample holder, consisting of a quartz monocrystal zero background plate. Diffraction data were collected applying the following conditions: angular range 2-40° 2$\theta$, 0.02°/step scan and acquisition time of 1s/step and 10s/step. The instrument calibration was verified by means of a NIST SRM 1976b. Data acquisition was performed by means of Bruker Diffraction Measurement Center software and data elaboration was performed by means of TOPAS software.

The XRPD patterns referred to nanocrystalline form of darolutamide are characterized by very broad peaks, with peak widths falling in many cases, well above 1.0° and an example of said pattern is depicted in Figure 1.

Example 16: Determination of crystal domain size

Crystal domain size calculation was performed applying Scherrer equation to the most isolated and clearly defined peaks in the X-ray diffraction patterns of NC form: the peaks falling near about 8.6 and 10.4 °2$\theta$.

To correctly determine the experimentally measurable peak widths (HE) of the very broad peaks listed above, a profile fitting (PF) technique with a "split pseudo-Voigt" description for the slightly asymmetric peak shapes was applied. See for the analytical descriptors of the different peak shapes (*e.g.* Gaussian, Lorentzian, Voigt, pseudo-Voigt, Pearson VII or split version) R. A. Young and D. B. Wiles, 'Peak shapes functions in Rietveld refinement', J. Appl. Cryst., 1982, 15, 430-438.

Figure 9 shows an example of profile fitting plot related to the peaks falling near 8.6 and 10.4° 2-Theta of nanocrystalline darolutamide.

The average value <HE> = ½ [HE(peak1) + HE(peak2)] was then computed, and corrected for instrumental aberration (with $H_I$ = 0.06 - 0.12°) into the average sample-dependent value <HS> using following equation:

$$< \mathrm{HS} >= \sqrt{< \mathrm{HE} >^{2} - H_{I}^{2}}$$

< HS > was then used to compute, through Scherrer Equation, the corresponding D values, one per each specimen.

As $H_I$ typically fall in the 0.06 - 0.12° range, any HE values above 0.3° does not need to be converted into $H_S$, therefore in said cases < HS > =< HE >

The Scherrer equation can be written as:

$$\mathrm{D} = \frac{\mathrm{k}\lambda}{\mathrm{<HS>}\ \cos\theta}$$

where:

D is the mean size of the ordered (crystalline) domains;
k is a dimensionless shape factor, with a value close to unity. The shape factor has a typical value of about 0.9, but varies with the actual shape of the crystallite;
$\lambda$ is the X-ray wavelength;
< HS > is the integral peak width, well approximated by the full width at half maximum (FWHM), corrected, if necessary, by subtracting the instrumental line broadening, in radians;
$\theta$ is the half of the $2\theta$ diffraction angle.

The calculated crystal domain size values for different batches of nanocrystalline form of darolutamide are reported in Table 6.

Table 6

| Darolutamide Sample from Example | HE of Peak 1 (at about 8.6°) | HE of Peak 2 (at about 10.3°) | <HS> [°] | Average D [nm] |
|---|---|---|---|---|
| Example 3 | 1.05 | 1.05 | 1.05 | 7.6 |
| Example 4 | 1.18 | 1.10 | 1.14 | 7.0 |
| Example 5 | 0.91 | 0.84 | 0.87 | 9.1 |
| Example 8 | 0.81 | 0.73 | 0.77 | 10.3 |
| Example 10 | 1.38 | 1.33 | 1.35 | 5.9 |
| Example 11 | 1.17 | 1.09 | 1.13 | 7.0 |

Example 17: Characterization by scanning electron microscope (SEM) images

Nanocrystalline form of darolutamide according to the present invention was analyzed by scanning electron microscope imaging. Figure 5 shows SEM images acquired at x 20000 fold magnification, scale bar 1 $\mu$m.

Nanocrystalline form of darolutamide prepared according to the present invention exhibits a complex framework composed by filaments which in turn are formed by other substructures with smaller size.

Example 18: DSC analysis

DSC analysis was carried out with a Mettler DSC-1 Star System, using 40 $\mu$L standard Aluminum crucibles loaded with about 3-6 mg of sample and sealed with a specific crucible sealing press. A void crucible of the same kind was placed in the reference location of the DSC furnace. Indium reference material was used to calibrate the apparatus with regard to the temperature scale and the enthalpy response. Samples were analyzed under nitrogen flow at a heating rate of 10°C/min after making a pinhole on the crucible lid; the explored thermal range was between 30 and 250°C.

A non-limiting example of DSC profile of Form NC is shown in Figure 2.

Example 19: $^{13}$C CPMAS NMR

The $^{13}$C CPMAS NMR spectra for all samples were acquired with a Jeol ECZR 600 instrument, operating at

600.17 and 150.91 MHz, respectively for $^{1}H$ and $^{13}C$ nuclei.

The powder samples were packed into cylindrical zirconia rotors with a 3.2 mm o.d. and a 60 μL volume. A certain amount of each sample was collected and used without further preparations to fill the rotor.

$^{13}C$ CPMAS NMR spectra were acquired at room temperature at a spinning speed of 20 kHz, using a ramp cross-polarization pulse sequence with a 90° 1H pulse of 2.0 μs, a contact time of 3.5 ms, an optimized recycle delay between 2.2 and 3.3 s, a number of scans in the range 800-1200, depending on the sample. For every spectrum, a two-pulse phase modulation (TPPM) decoupling scheme was used, with a radiofrequency field of 108.5 kHz. The 13C chemical shift scale was calibrated through the methylenic signal of external standard glycine (at 43.7 ppm).

Table 7 shows the chemical shift values (in ppm) of the peaks observed in the $^{13}C$ CPMAS NMR spectra of a characteristic samples of nanocrystalline form of darolutamide.

Table 7

| **$^{13}C$ CPMAS peaks [ppm]** |
|---|
| 163.3 |
| 162.4 |
| 152.1 |
| **148.0** |
| **139.2** |
| 134.1 |
| 126.2 |
| 122.5 |
| 115.0 |
| 111.1 |
| 107.3 |
| 104.8 |
| 102.4 |
| 62.2 |
| 57.4 |
| **47.4** |
| **19.5** |

An example of $^{13}C$ CPMAS NMR spectra of nanocrystalline form of darolutamide is shown in Figure 3.

Example 20: stability study

5 aliquots of about 1 g of nanocrystalline form of present invention were stored at the following conditions wherein RH is referred to relative humidity :

- 40 °C and 75% RH, double PE bag in HDPE bottle

The sample were analysed before the storage ($T_0$), after 1 month, 3 months , 6 months and 12 months using high performance chromatography (HPLC) method to control the purity and X-ray powder diffractogram (XRPD) to evaluate any change in crystalline form.

The HPLC analysis show that nanocrystalline form of the present invention does not present a significant worsening of the purity, in terms of formation of new impurities and increase of the content of those already present profile with respect to the initial profile acquired at $T_0$. The X-ray powder diffractograms (XRPD) recorded at the reported timepoints showed no signs of transformation into a different crystalline form as reported in Figure 10.

Example 21: Identification of crystalline form of darolutamide contained in Nubeqa®

Nubeqa® 300 mg drug product was compared by means of X-ray powder diffractogram (XRPD) with darolutamide Form I, obtained according to the teachings of WO 2016/120530. See Figure 11. The analysed sample of Nubequa® 300 mg was prepared by means of grinding a drug product tablet in an agate mortar to obtain a suitable powder sample for XRPD sampling.

The comparison of the presented profiles allowed detecting characteristic Form I reflections at 8.5, 10.4, 16.6,

16.9, and 24.3 ± 0.15 degrees 2-Theta in Nubeqa® 300 mg drug product XRPD pattern when measured at room temperature with Cu-Kα radiation having a wavelength of 1.5419 Å.

Example 22: Tablet preparation

Tablets of nanocrystalline form of darolutamide were prepared using the ingredients list reported in Table 8 here below.

Table 8

| | [%] |
|---|---|
| Darolutamide NC | 42.0% |
| Lactose monohydrate | 35.0% |
| Calcium hydrogen phosphate (anhydrous) | 14.1% |
| Sodium croscarmellose | 1.2% |
| Povidone | 5.7% |
| Magnesium stearate | 2.0% |
| Purified water | |
| SUM | 100% |

All components, with the exception of magnesium stearate and with only a portion of croscarmellose sodium in a high-shear granulator were mixed. Water was sprayed into the mixture to do the granulation. Granules were dried in a fluid bed dryer, then the rest of the croscarmellose sodium and magnesium stearate were added followed by mixing.

The obtained mixture was compressed in a tableting machine.

Optionally, tablets may be coated using conventional coating agent until a weight increase of about 3%, more preferably using as coating materials polyvinyl alcohol iron oxide, talc, and titanium dioxide.

Example 23: Preparation of nanocrystalline form of darolutamide starting from a darolutamide solution obtained directly from the reduction of 3-acetyl-N-[(2S)-1-[3-(3-chloro-4-cyanophenyl)pyrazol-1-yl]propan-2-yl]-1H-pyrazole-5-carboxamide (Ketodarolutamide)

In a glass reactor 90 g (227 mmol) of Ketodarolutamide, 540 mL of 2-methyltetrahydrofuran, 45 mL of water were added. A suspension of $NaBH_4$ (8.60 g, 227 mmol) in 2-methyltetrahydrofuran was added in four portions at room temperature and the mixture was stirred at the same temperature for 4 hours. 1N HCl was added up to pH about 2, the mixture was stirred for about 30 minutes at 20-30°C, then the aqueous phase was discarded and the organic phase was diluted with 2-methyltetrahydrofuran (180 ml) and washed two times with water (180 mL per time).

The resulting water content was about 12% measured by KF analysis. The organic phase was filtered through celite and diluted with further 2-methyltetrahydrofuran (450 mL) used to wash the pad. Then the solution was cooled to 5/15°C and n-heptane was added (360 mL), followed by a seed of darolutamide Form NC (2.7 g). The mixture was cooled to - 5/+5°C and n-heptane (990 mL) was added dropwise in about 2 hours while keeping the temperature below +5°C.

The resulting suspension was stirred for 3 hours maintaining the temperature at -5/+5°C, then the solid was recovered by filtration. The solid cake was washed with n-heptane (270 mL) and the wet solid was dried in a vacuum oven at 50°C for about 16 hours. Darolutamide Form NC was obtained (89.5 g). The Specific Surface Area (SSA) of this sample measured as described above in Example 13 was 46.0 $m^2/g$.

Example 24: Preparation of nanocrystalline form of darolutamide starting from darolutamide in a solid form

Darolutamide (90 g) was dissolved in a mixture of 2-methyltetrahydrofuran (1080 mL) and water (180 ml) by stirring at 20/30°C in a glass reactor. The bottom aqueous layer was separated and discarded, and the remaining organic solution was filtered through a celite pad, which was washed with 2-methyltetrahydrofuran (270 mL) then combined with the rest of the solution. The solution was cooled to 5/15°C and n-heptane was added (360 mL), followed by a seed of darolutamide Form NC (2.7 g). The mixture was cooled to -5/+5°C and n-heptane (990 mL) was added dropwise in about 2 hours while keeping the temperature below +5°C.

The resulting suspension was stirred for 6 hours maintaining the temperature at -5/+5°C, then the solid was recovered by filtration. The solid cake was washed with n-heptane (270 mL) and the wet solid was dried in a vacuum oven

at 50°C for about 16 hours. Darolutamide Form NC was obtained (88 g). The Specific Surface Area (SSA) of this sample measured as described above in Example 13 was 43.8 $m^2/g$.

**Citation List**

Patent Literature:

- WO 2011/051540
- WO 2016/120530
- WO 2018/162793
- EP 3592732
- EP 3495352


Non Patent Literature:

- B. Movahedi, "Introductory Chapter: Nanocrystalline Materials", Nanocrystalline Materials, Feb. 2020, doi: 10.5772/intechopen.90255
- Nasrollahzadeh, M. et al., "Chapter 2 - Types of Nanostructures" "Interface Science and Technology", Volume 28, Chapter 2; 2019, Pages 29-80;
- Suryanarayana, C., "Nanocrystalline Materials", International Materials Reviews, 1995, 40(2):41-64
- U. Holtzward and N.Gibson 'The Scherrer equation versus the 'Debye-Scherrer equation', Nature Nanotechnology, 2011, 6, 534.
- Kyung Rok Chu et al. Effect of particle size on the dissolution behaviours of poorly water-soluble drugs, Arch. Pharm. Res., 2012 July, 35(7), 1187-95, DOI 10.1007/s 12272-012-0709-3


For reasons of completeness, various aspects of the invention are set out in the following numbered clauses:

Clause 1. A nanocrystalline form of N-[(2S)-1-[3-(3-chloro-4-cyanophenyl)-1H-pyrazol-1-yl]-propan-2-yl]-5-(1-hydroxyethyl)-1H-pyrazole-3-carboxamide (darolutamide), characterized by having a specific surface area (SSA) in the range from 40 $m^2/g$ to 150 $m^2/g$, preferably from about 50 $m^2/g$ to about 135 $m^2/g$, more preferably from 60 $m^2/g$ to 135 $m^2/g$, measured by the multi-point nitrogen adsorption technique based on the Brunauer, Emmett and Teller (BET) theory.

Clause 2. The nanocrystalline form according to clause 1 characterized by having a powder X-ray diffractogram comprising broad peaks at 10.2, 14.4, and 17.8 $\pm$ 0.2 degrees 2-Theta, when measured at room temperature with Cu-K$\alpha$ radiation having a wavelength of 1.5419 Å.

Clause 3. The nanocrystalline form according to clause 2 characterized by having a powder X-ray diffractogram additionally comprising each of the broad peaks at of 8.6 and 23.6 $\pm$ 0.2 degrees 2-Theta and at least one of the following broad peaks at of 6.2, 16.3, and 22.2 $\pm$ 0.2 degrees 2-Theta, when measured at room temperature with Cu-K$\alpha$ radiation having a wavelength of 1.5419 Å.

Clause 4. The nanocrystalline form according to any one of the preceding clauses characterized by having a crystallite size comprising from 5 to 11 nm, preferably from 6 nm to 8 nm, as determined by Scherrer equation.

Clause 5. The nanocrystalline form according to any one of the preceding clauses characterized by having DSC curve comprising a broad endothermic transition with a peak temperature enclosed in the range 130- 150 °C, when measured with DSC at a heating rate of 10 °C/min.

Clause 6. The nanocrystalline form according to any one of the preceding clauses characterized by having a $^{13}C$ CPMAS NMR spectrum comprising peaks at 163.3 $\pm$ 0.2 ppm, 107.3 $\pm$ 0.2 ppm, 102.4 $\pm$ 0.2 ppm, and 57.4 $\pm$ 0.2 ppm.

Clause 7. A process for the preparation of the nanocrystalline form as defined in any one of the preceding clauses, said process comprising the steps of:

(i) providing a solution of darolutamide in an organic solvent selected from the group consisting of 2-methyltetrahydrofuran, tetrahydrofuran, dioxane, and any mixture thereof, said solution containing an amount of water between 2 % to 7 % by weight of the total solution, measured according to Karl Fischer method;
(ii) optionally filtering;

(iii) cooling the obtained solution to a temperature from -5 °C to 10 °C;
(iv) optionally seeding;
(v) adding an antisolvent selected from the group consisting of C5-C7 linear or branched alkane, cycloalkane, substituted cycloalkane, toluene, symmetric ethers, and asymmetric ethers, characterized in that the temperature of the mixture is maintained in the range of from -5 °C to 10 °C during the addition;
(vi) optionally adding other portions of the antisolvent used in step (v) to cause the complete precipitation of the solid;
(vii) recovering the resulting solid and, optionally, drying it.

Clause 8. The process according to clause 7 wherein darolutamide concentration of the solution in step (i) usually ranges from 160 to 60 g/L, preferably from 145 to 135 g/L. Clause 9. The process according to clauses 7 and 8, wherein step (i) is performed in 2-methyltetrahydrofuran in a range of temperature from 25 °C to 70 °C.
Clause 10. The process according to any of the clauses 7 to 9, wherein the antisolvent-solvent ratio is between from 1:1 to 3:1, preferably 2:1, said ratio based on the organic solvent volume present in the solution provided in step (i).
Clause 11. The process according to any of the clauses 7 to 10, wherein the antisolvent is selected from C5-C7 linear alkane, preferably heptane, and toluene, more preferably the antisolvent is heptane.
Clause 12. The process according to clause 7 wherein the solution of step (i) is obtained following the steps comprising:

(viii) providing darolutamide dissolved in a mixture of an organic solvent selected from the group consisting of 2-methyltetrahydrofuran, tetrahydrofuran, dioxane, and any mixture thereof, and water, wherein the water amount is upper to 7 % by weight of the total mixture, measured according to Karl Fischer method;
(ix) decanting the phases and discharging the aqueous layer;
(x) distilling the organic solvent at ambient pressure until a residual solution having a darolutamide concentration from 160 to 60 g/L is obtained;
(xi) optionally adding another portion of fresh organic solvent or mixture thereof selected from the organic solvents used in step (viii) and performing step (x), in order to obtain a solution containing an amount of water between 2 % to 7 % by weight of the total solution, measured according to Karl Fischer method.

Clause 13. The process according to clause 12 wherein step (viii) is performed using a solution obtained directly from the organic layer arising from the work-up of a reaction that lead to obtain darolutamide as product.
Clause 14. The process according to clause 13, wherein the reaction is a reduction process of ketodarolutamide (II) carried out in a mixture of an organic solvent selected from the group consisting of 2-methyltetrahydrofuran, tetrahydrofuran, dioxane, and a mixture thereof, and water, in the presence of a sodium borohydride as a reductive agent.
Clause 15. Use of the nanocrystalline form as defined in any one of clauses 1 to 6 for the "preparation of a pharmaceutical composition.
Clause 16. A pharmaceutical composition comprising the nanocrystalline form as defined in any one of the clauses 1 to 6 and at least one pharmaceutically acceptable excipient.

## Claims

1. A nanocrystalline form of N-[(2S)-1-[3-(3-chloro-4-cyanophenyl)-1H-pyrazol-1-yl]-propan-2-yl]-5-(1-hydroxyethyl)-1H-pyrazole-3-carboxamide (darolutamide) each particle of said form being composed by crystallites having a size below 100 nm, **characterized by** having a powder X-ray diffractogram comprising broad peaks at 10.2, 14.4, and 17.8 $\pm$ 0.2 degrees 2-Theta, when measured at room temperature with Cu-K$\alpha$ radiation having a wavelength of 1.5419 Å.

2. The nanocrystalline form according to claim 1 **characterized by** having a powder X-ray diffractogram additionally comprising each of the broad peaks at of 8.6 and 23.6 $\pm$ 0.2 degrees 2-Theta and at least one of the following broad peaks at of 6.2, 16.3, and 22.2 $\pm$ 0.2 degrees 2-Theta, when measured at room temperature with Cu-K$\alpha$ radiation having a wavelength of 1.5419 Å.

3. The nanocrystalline form according to claim 1 **characterized by** X-ray diffraction pattern shown in Figure 1.

4. The nanocrystalline form according to any one of the preceding claims **characterized by** having DSC curve comprising a broad endothermic transition with a peak temperature enclosed in the range 130-150 °C, when

measured with DSC at a heating rate of 10 °C/min.

5. The nanocrystalline form according to any one of the preceding claims **characterized by** having a $^{13}C$ CPMAS NMR spectrum comprising peaks at 163.3 ± 0.2 ppm, 107.3 ± 0.2 ppm, 102.4 ± 0.2 ppm, and 57.4 ± 0.2 ppm.

6. A process for the preparation of the nanocrystalline form as defined in any one of the preceding claims, said process comprising the steps of:

   (i) providing a solution of darolutamide in an organic solvent selected from the group consisting of 2-methyltetrahydrofuran, tetrahydrofuran, dioxane, and any mixture thereof, said solution containing an amount of water between 2 % to 20 % by weight of the total solution;
   (ii) optionally filtering;
   (iii) cooling the obtained solution to a temperature from -5 °C to 15 °C;
   (iv) optionally seeding;
   (v) adding an antisolvent selected from the group consisting of $C_5$-$C_7$ linear or branched alkane, cycloalkane, substituted cycloalkane, toluene, symmetric ethers, and asymmetric ethers, **characterized in that** the temperature of the mixture is maintained in the range of from -5 °C to 15 °C during the addition;
   (vi) optionally adding other portions of the antisolvent used in step (v) to cause the complete precipitation of the solid;
   (vii) recovering the resulting solid and, optionally, drying it.

7. The process according to claim 6 wherein darolutamide concentration of the solution in step (i) usually ranges from 160 to 30 g/L, preferably from 145 to 45 g/L.

8. The process according to claims 6 and 7, wherein step (i) is performed in 2-methyltetrahydrofuran in a range of temperature from 25 °C to 70 °C.

9. The process according to any of the claims 6 to 8, wherein the antisolvent-solvent ratio is between from 1:1 to 3:1, preferably 2:1, said ratio based on the organic solvent volume present in the solution provided in step (i).

10. The process according to any of the claims 6 to 9, wherein the antisolvent is selected from $C_5$-$C_7$ linear alkane, preferably heptane, and toluene, more preferably the antisolvent is heptane.

11. The process according to claim 6 wherein the solution of step (i) is obtained following the steps comprising:

    (viii) providing darolutamide dissolved in a mixture of an organic solvent selected from the group consisting of 2-methyltetrahydrofuran, tetrahydrofuran, dioxane, and any mixture thereof, and water, wherein the water amount is up to 20 % by weight of the total mixture;
    (ix) if two phases are present, decanting the phases and discharging the aqueous layer;
    (x) optionally, distilling the organic solvent at ambient pressure until a residual solution having a darolutamide concentration from 160 to 30 g/L is obtained;
    (xi) optionally adding another portion of fresh organic solvent or mixture thereof selected from the organic solvents used in step (viii) and performing step (x), in order to obtain a solution containing an amount of water between 2 % to 20 % by weight of the total solution.

12. The process according to claim 11 wherein step (viii) is performed using a solution obtained directly from the organic layer arising from the work-up of a reaction that lead to obtain darolutamide as product.

13. The process according to claim 12, wherein the reaction is a reduction process of ketodarolutamide (II) carried out in a mixture of an organic solvent selected from the group consisting of 2-methyltetrahydrofuran, tetrahydrofuran, dioxane, and a mixture thereof, and water, in the presence of a sodium borohydride as a reductive agent.

14. Use of the nanocrystalline form as defined in any one of claims 1 to 5 for the preparation of a pharmaceutical composition.

15. A pharmaceutical composition comprising the nanocrystalline form as defined in any one of the claims 1 to 5 and at least one pharmaceutically acceptable excipient.

Fig. 1

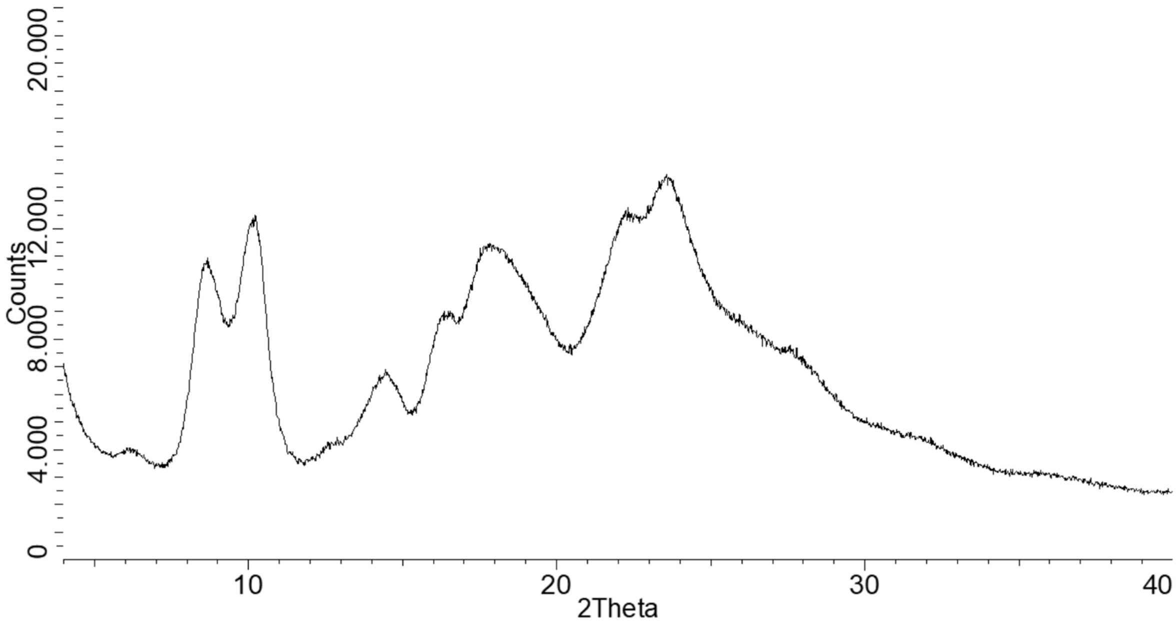


Fig. 2

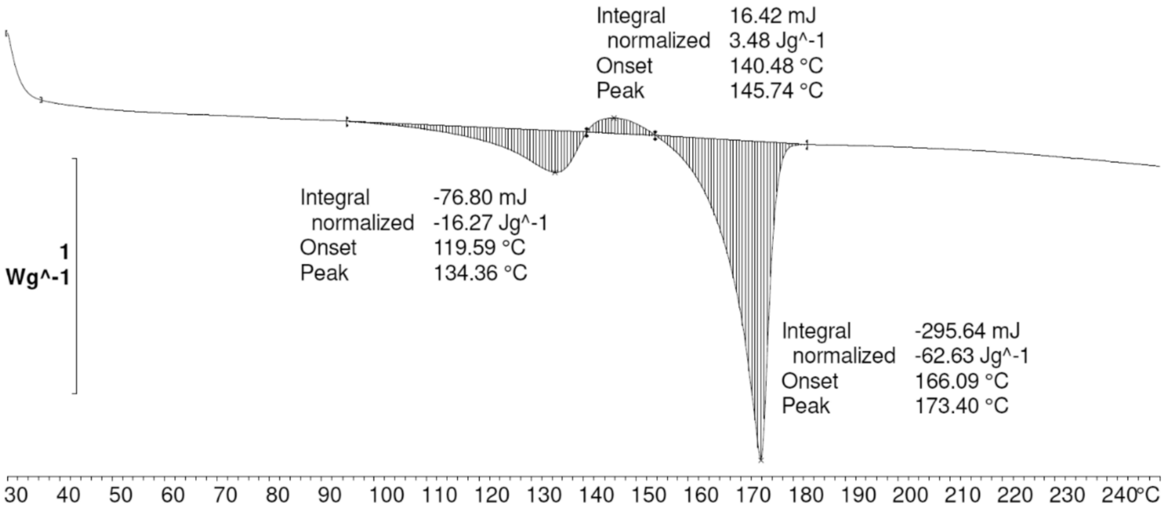


Fig. 3

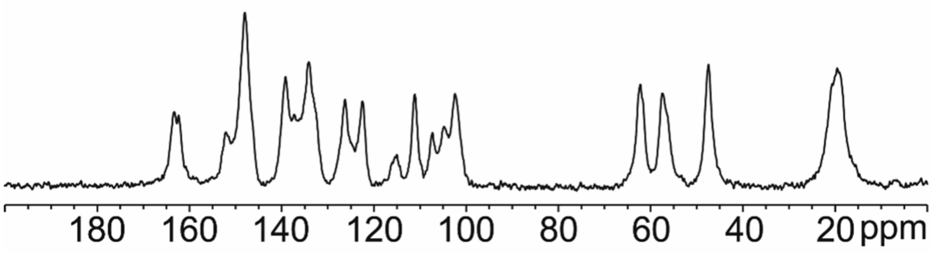

Fig. 4

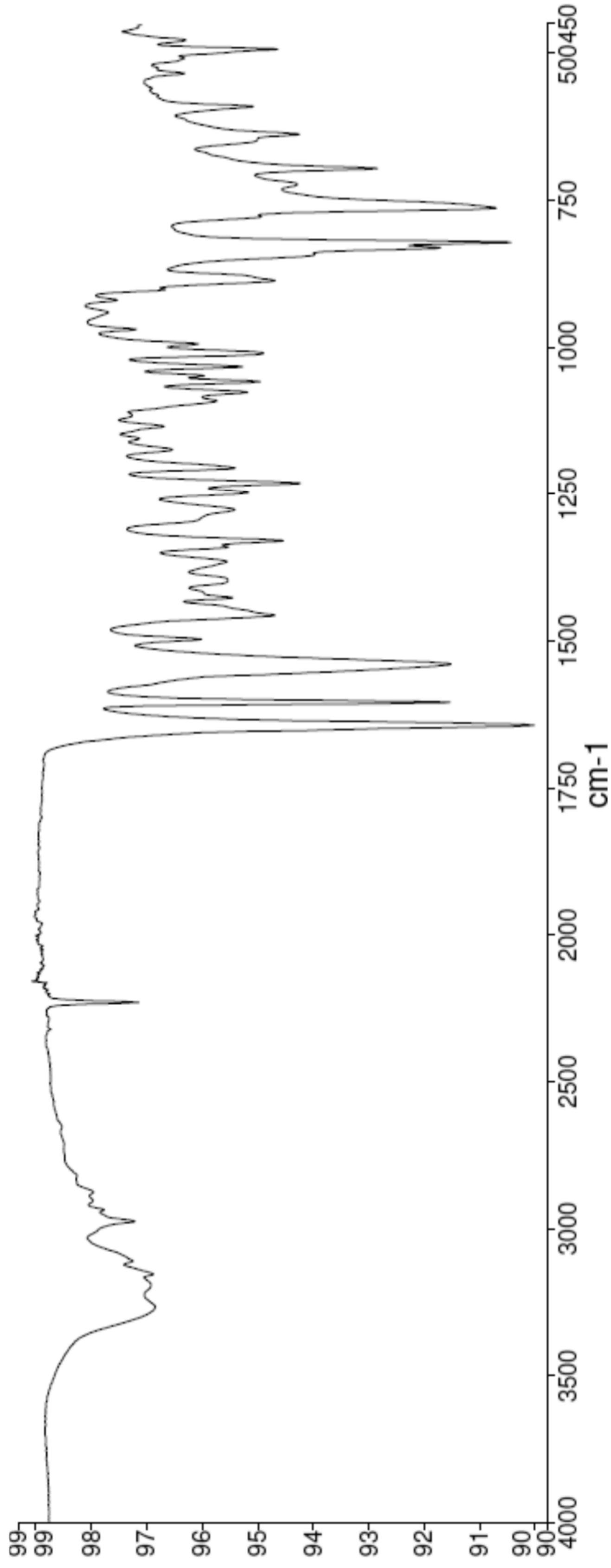
cm-1
4000
3500
3000
2500
2000
1750
1500
1250
1000
750
500450
99
98
97
96
95
94
93
92
91
90

Fig. 5

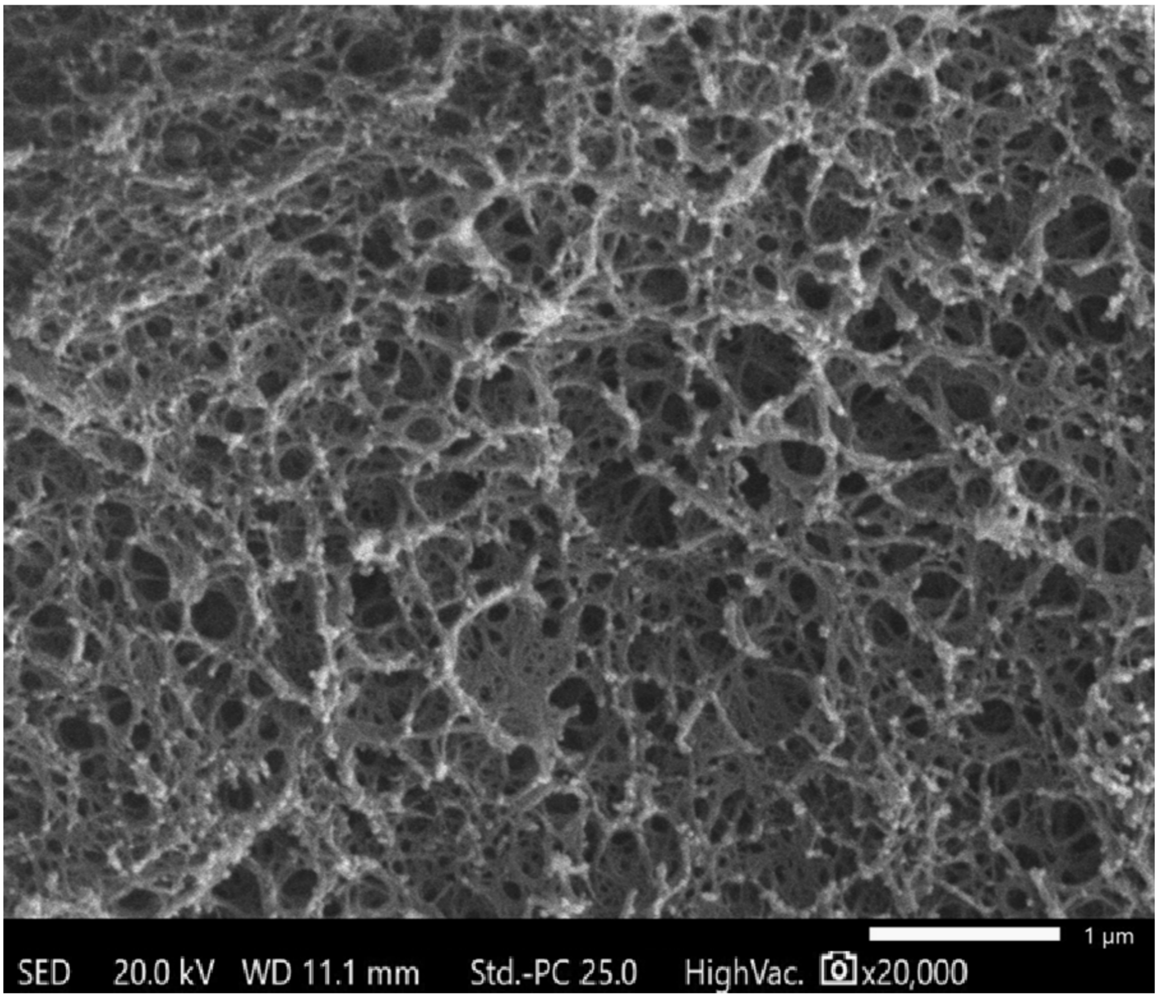

Fig. 6

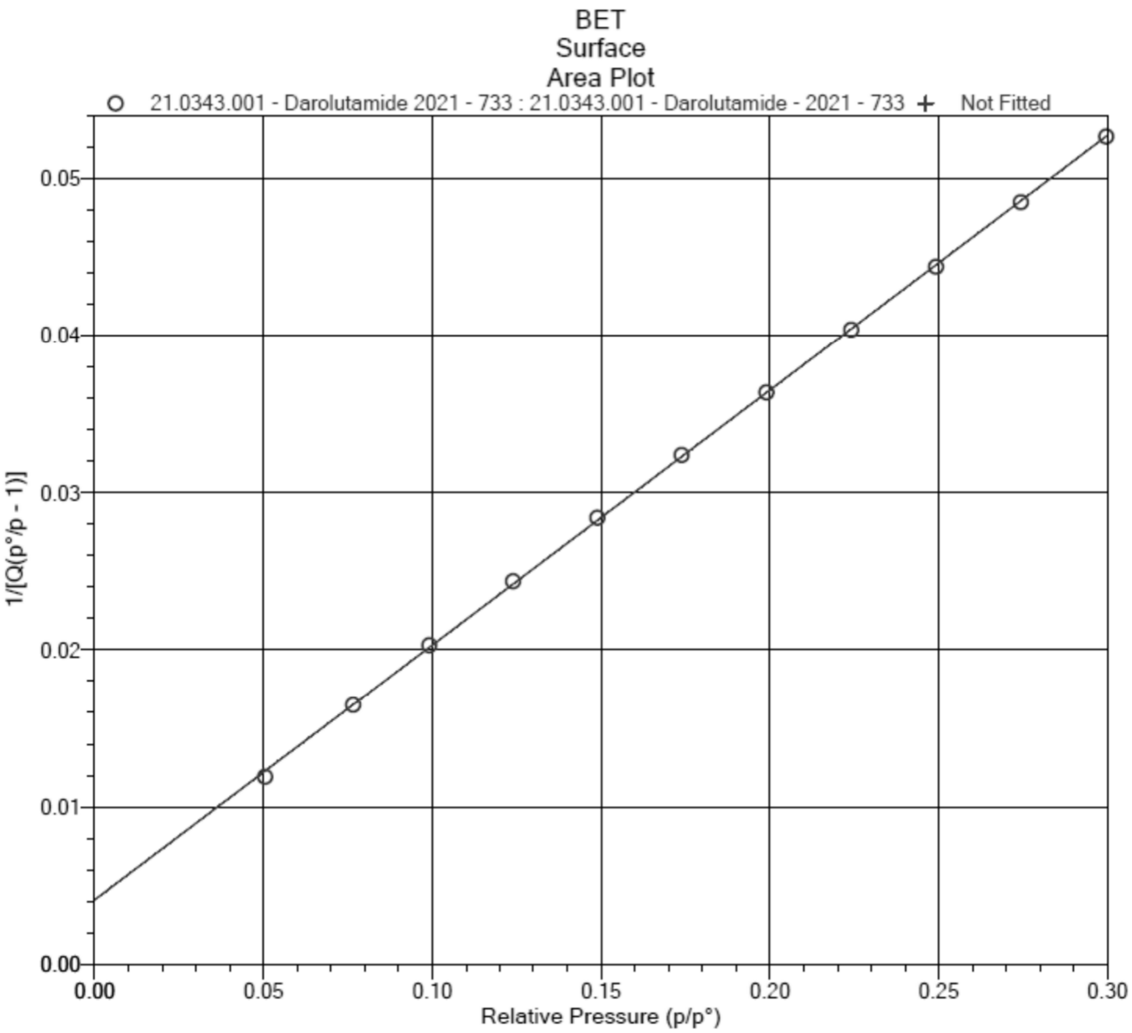
BET
Surface
Area Plot
21.0343.001 - Darolutamide 2021 - 733 : 21.0343.001 - Darolutamide - 2021 - 733
Not Fitted
0.05
0.04
0.03
0.02
0.01
0.00
1/[Q(p°/p - 1)]
0.00
0.05
0.10
0.15
0.20
0.25
0.30
Relative Pressure (p/p°)

Fig. 7

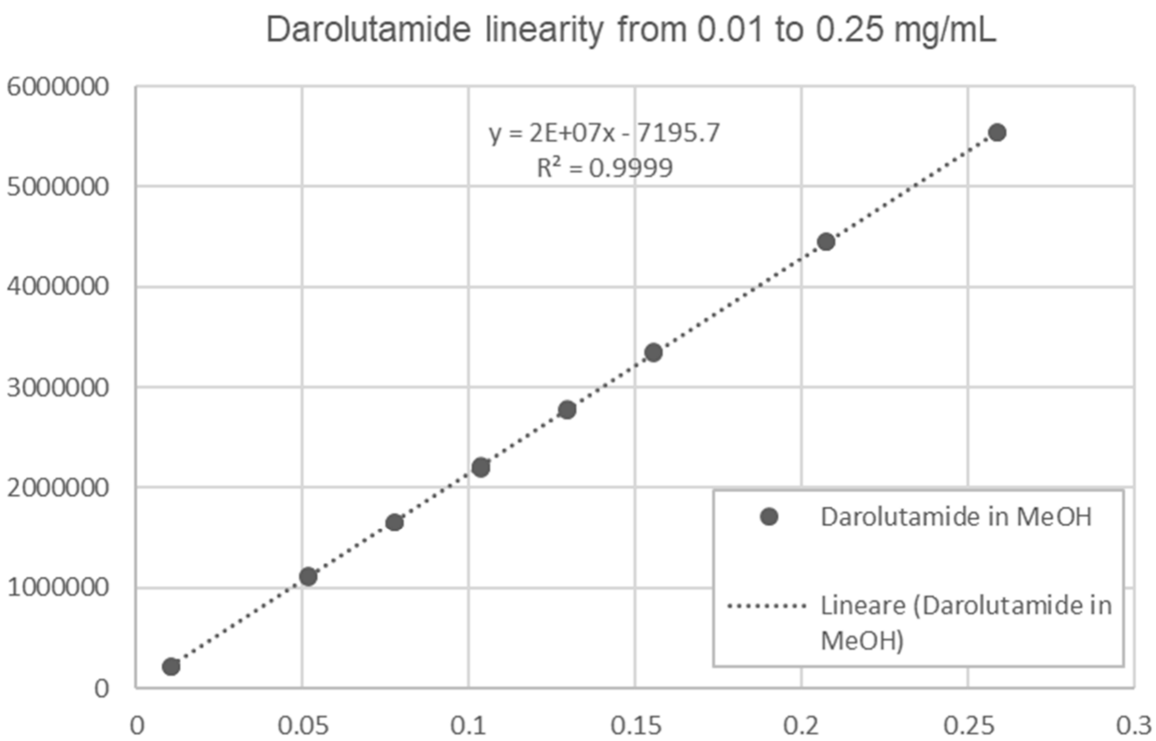
Darolutamide linearity from 0.01 to 0.25 mg/mL
y = 2E+07x - 7195.7
R² = 0.9999
6000000
5000000
4000000
3000000
2000000
1000000
0
0
0.05
0.1
0.15
0.2
0.25
0.3
Darolutamide in MeOH
Lineare (Darolutamide in MeOH)

Fig. 8

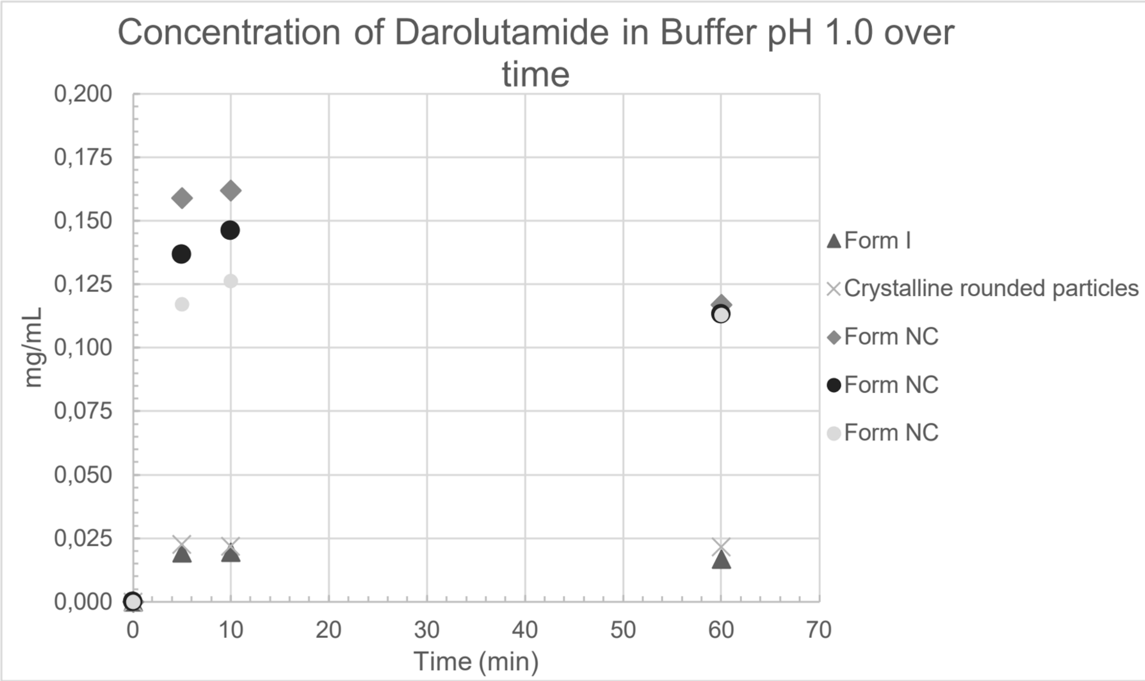
Concentration of Darolutamide in Buffer pH 1.0 over time
0,200
0,175
0,150
0,125
0,100
0,075
0,050
0,025
0,000
mg/mL
0
10
20
30
40
50
60
70
Time (min)
Form I
Crystalline rounded particles
Form NC
Form NC
Form NC

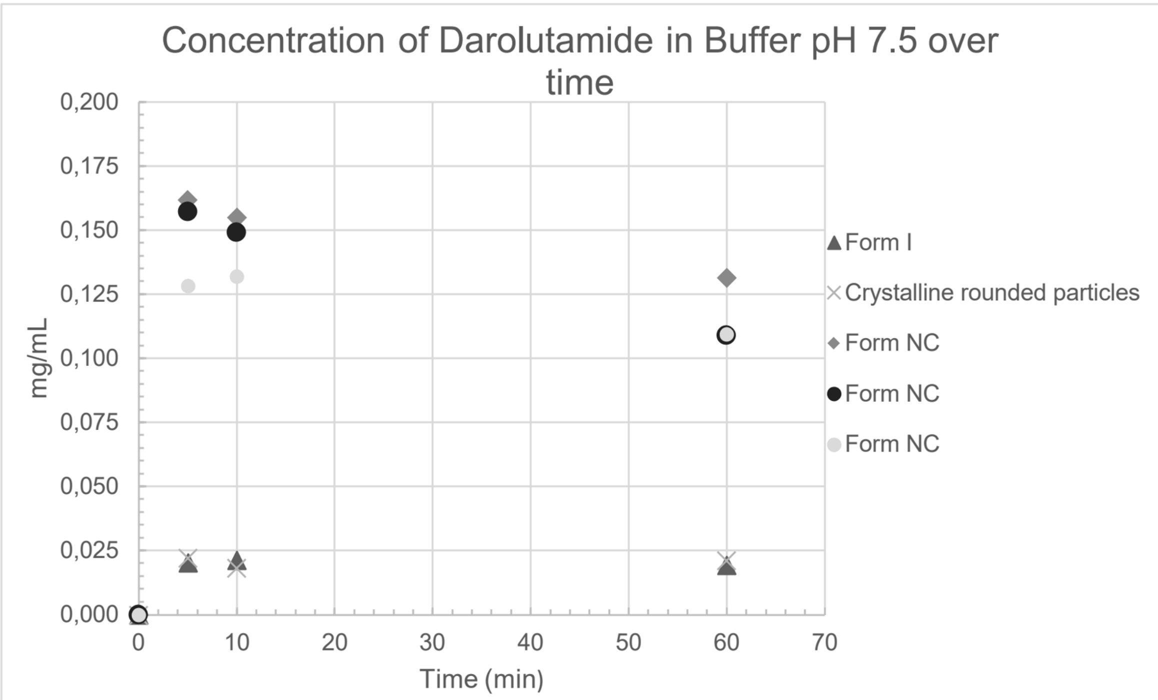
Concentration of Darolutamide in Buffer pH 7.5 over time
0,200
0,175
0,150
0,125
0,100
0,075
0,050
0,025
0,000
mg/mL
0
10
20
30
40
50
60
70
Time (min)
Form I
Crystalline rounded particles
Form NC
Form NC
Form NC

Fig. 9

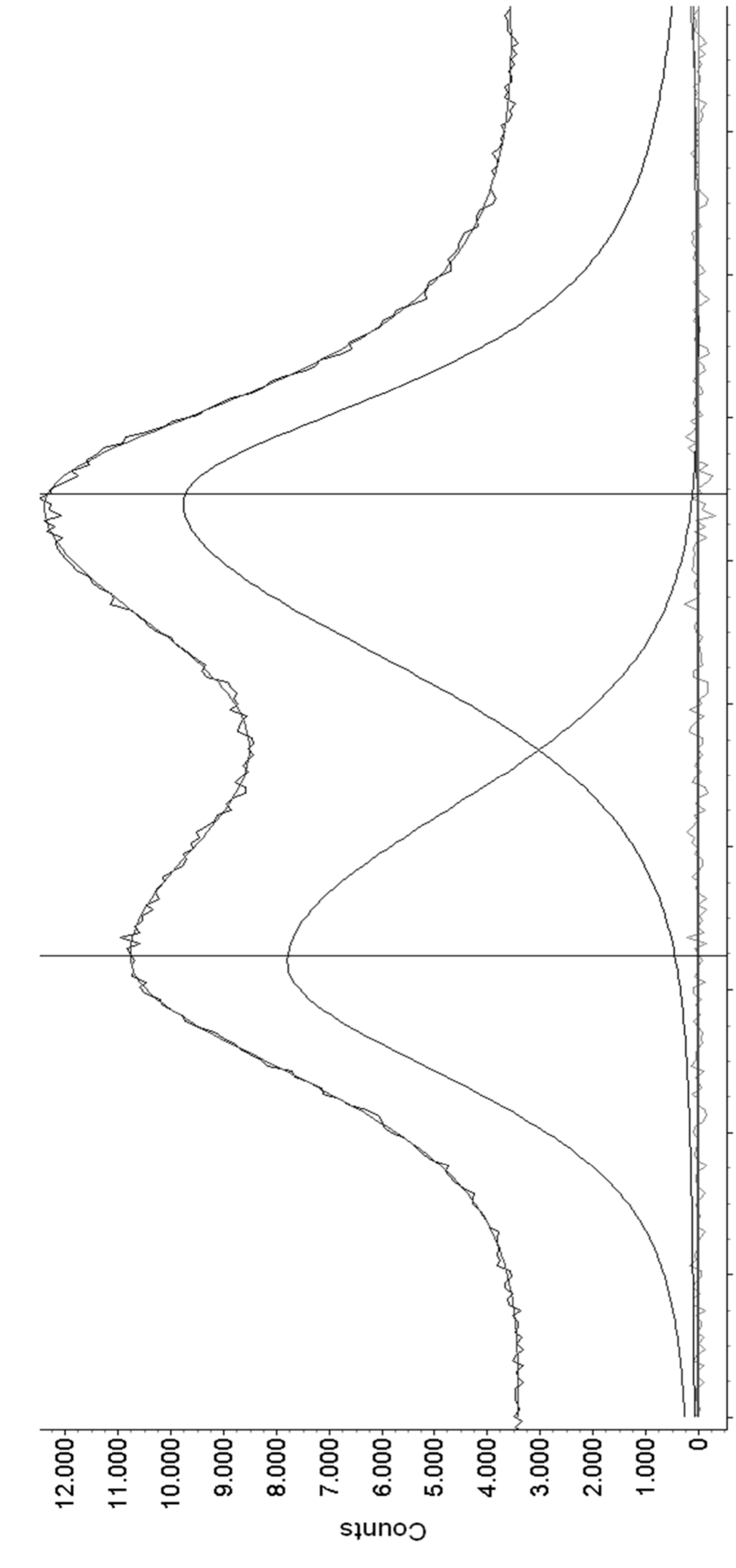
Counts
12.000
11.000
10.000
9.000
8.000
7.000
6.000
5.000
4.000
3.000
2.000
1.000
0
7
7,5
8
8,5
9
9,5
10
10,5
11
11,5
2Th Degrees

Fig. 10

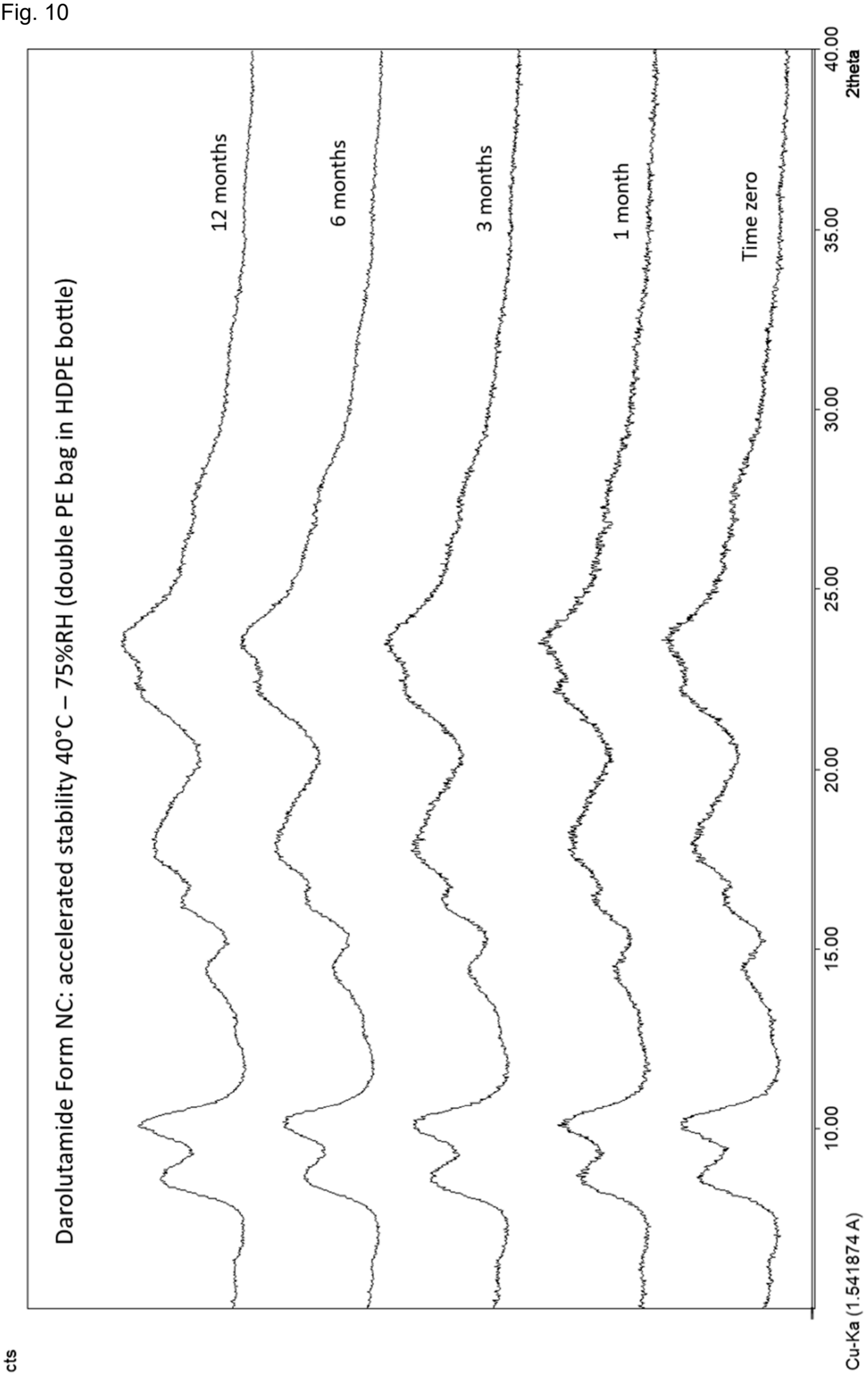
Darolutamide Form NC: accelerated stability 40°C – 75%RH (double PE bag in HDPE bottle)
12 months
6 months
3 months
1 month
Time zero
10.00
15.00
20.00
25.00
30.00
35.00
40.00
2theta
Cu-Ka (1.541874 A)
cts

Fig. 11

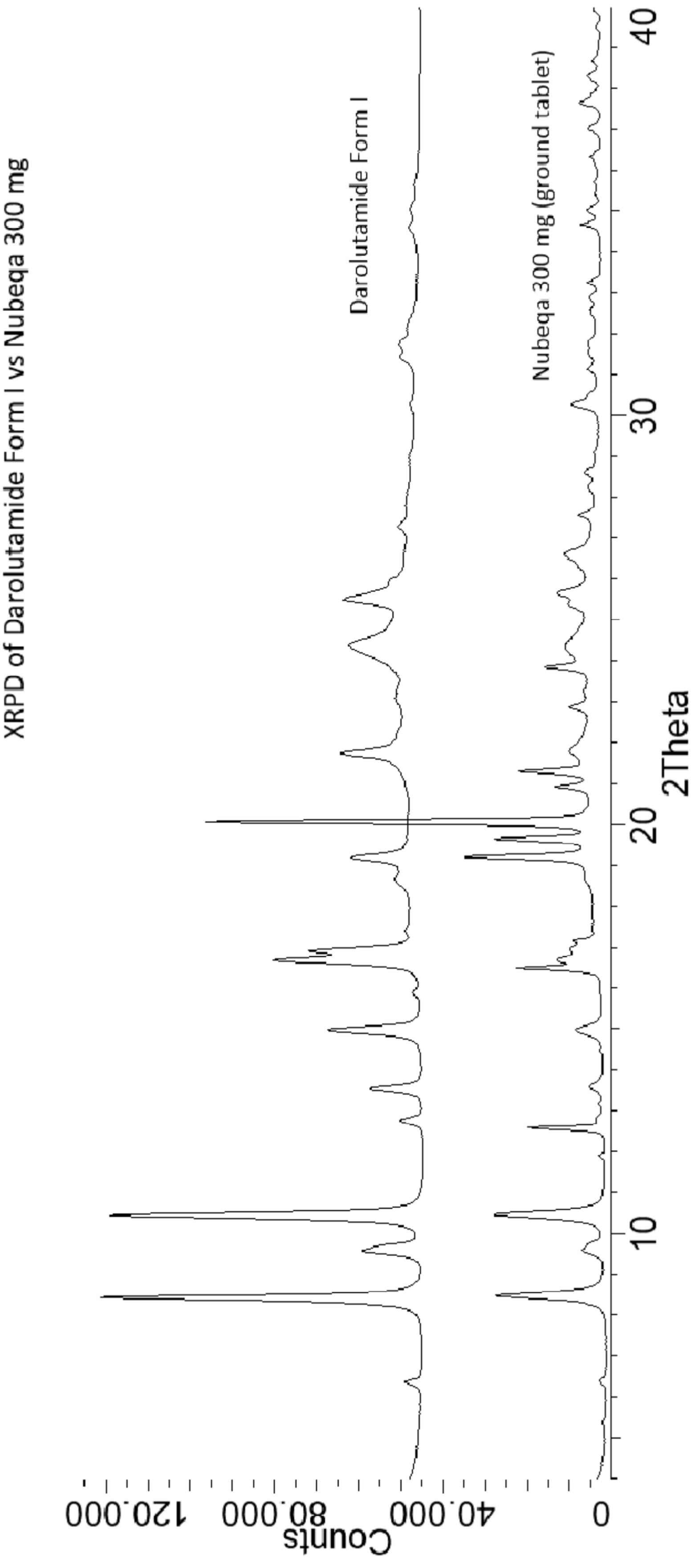
XRPD of Darolutamide Form I vs Nubeqa 300 mg
Darolutamide Form I
Nubeqa 300 mg (ground tablet)
Counts
0
40.000
80.000
120.000
2Theta
10
20
30
40

Fig. 12

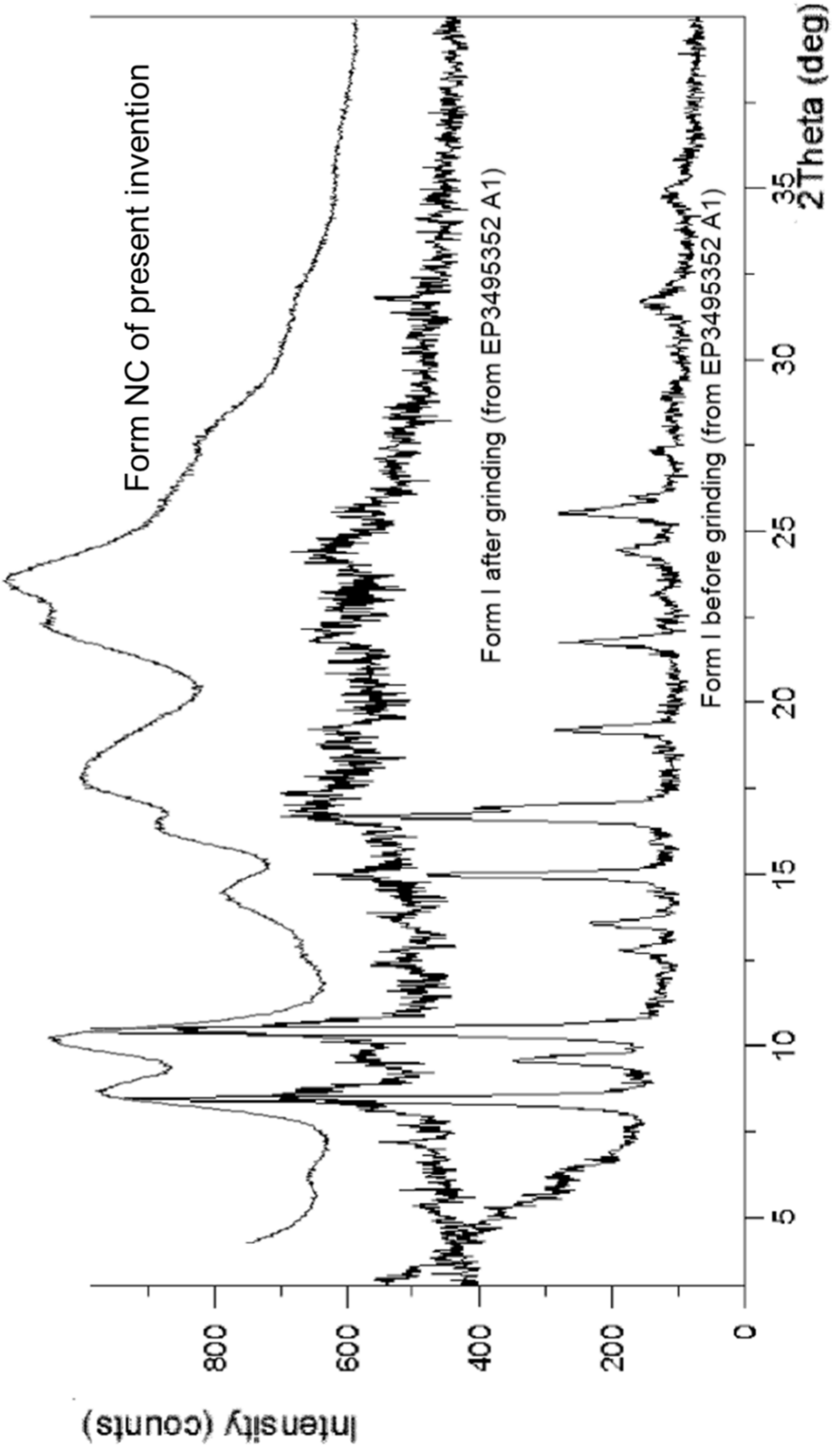
Form NC of present invention
Form I after grinding (from EP3495352 A1)
Form I before grinding (from EP3495352 A1)
2Theta (deg)
5
10
15
20
25
30
35
Intensity (counts)
0
200
400
600
800

Fig.13

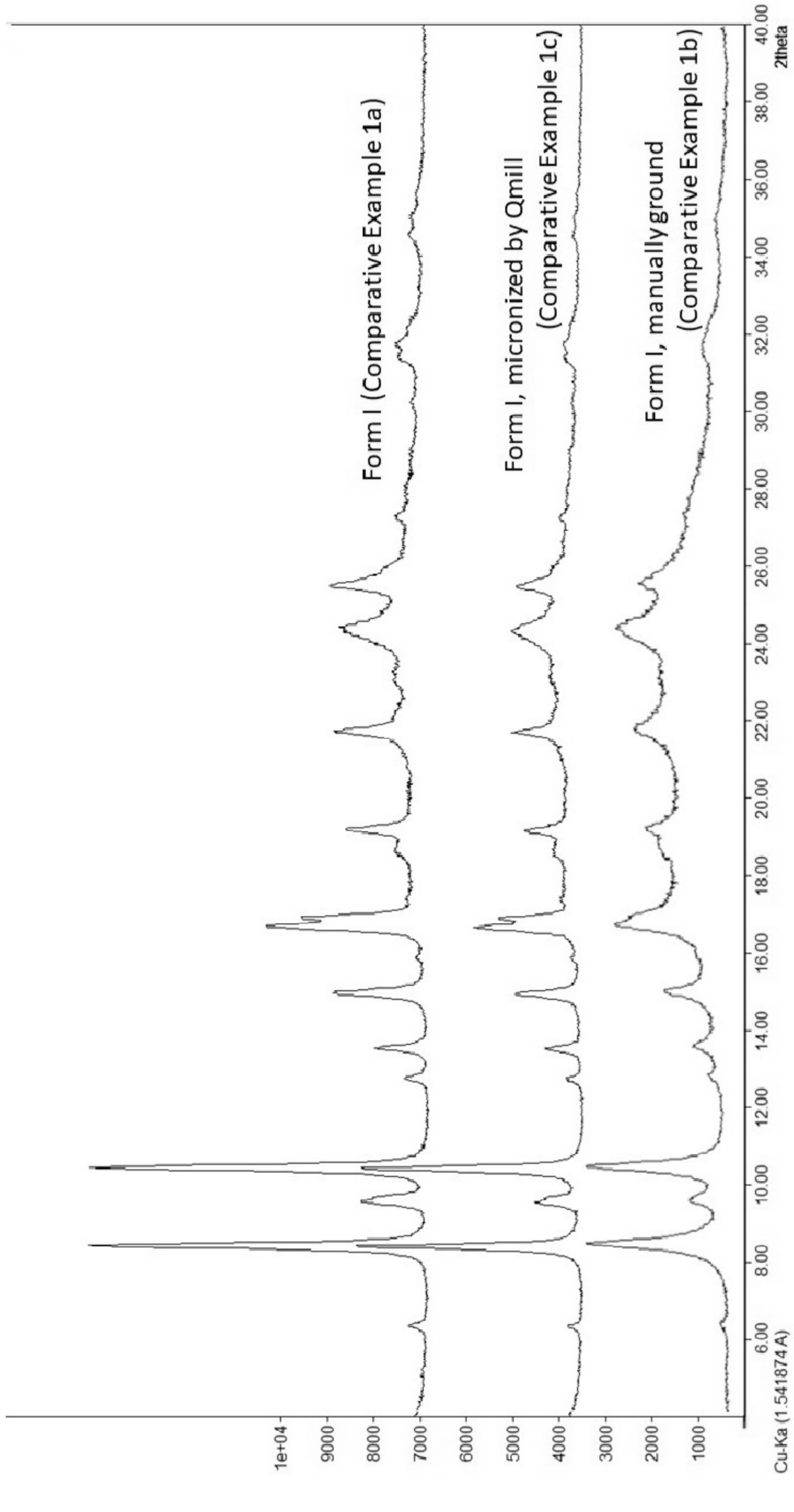
Form I (Comparative Example 1a)
Form I, micronized by Qmill
(Comparative Example 1c)
Form I, manuallyground
(Comparative Example 1b)
2theta
Cu-Ka (1.541874 A)
1e+04
9000
8000
7000
6000
5000
4000
3000
2000
1000
6.00
8.00
10.00
12.00
14.00
16.00
18.00
20.00
22.00
24.00
26.00
28.00
30.00
32.00
34.00
36.00
38.00
40.00

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011051540 A
- WO 2016120530 A
- WO 2018162793 A
- EP 3592732 A
- EP 3495352 A1
- EP 3495352 A


### Non-patent literature cited in the description


- **B. MOVAHEDI**. Introductory Chapter: Nanocrystalline Materials. *Nanocrystalline Materials*, February 2020
- Types of Nanostructures. **NASROLLAHZADEH, M. et al.** Interface Science and Technology. 2019, vol. 28, 29-80
- **SURYANARAYANA, C.** Nanocrystalline Materials. *International Materials Reviews*, 1995, vol. 40 (2), 41-64
- **U. HOLTZWARD** ; **N.GIBSON**. The Scherrer equation versus the 'Debye-Scherrer equation. *Nature Nanotechnology*, 2011, vol. 6, 534
- **KYUNG ROK CHU et al.** Effect of particle size on the dissolution behaviours of poorly water-soluble drugs. *Arch. Pharm. Res.*, 03 July 2012, vol. 5 (7), 1187-95
- **R. A. YOUNG** ; **D. B. WILES**. Peak shapes functions in Rietveld refinement. *J. Appl. Cryst.*, 1982, vol. 15, 430-438
- Nanocrystalline Materials. **B. MOVAHEDI**. Nanocrystalline Materials. February 2020
- **KYUNG ROK CHU et al.** Effect of particle size on the dissolution behaviours of poorly water-soluble drugs. *Arch. Pharm. Res.*, July 2012, vol. 35 (7), 1187-95